# EUROPEAN PATENT APPLICATION

(11) **EP 4 112 632 A1**
(43) Date of publication of application: **04.01.2023**
(21) Application number: 22150399.8
(22) Date of filing: 06.01.2022
(51) Int. Cl.: C07K 14/00, G16B 15/20, C07K 19/00

(54) **SYMMETRIC PROTEINS**

(30) Priority: 02.07.2021 EP 21183379
(71) Applicant: Katholieke Universiteit Leuven, 3000 Leuven (BE)
(72) Inventor: The designation of the inventor has not yet been filed

(57) **Abstract**

The invention relates to protein building block named the Self-Assembling Kelch (SAKe) protein. The protein has a stable, symmetric design with readily accessible loops that can be varied in both sequence and length to later bind larger molecules or scaffold a catalytic site.

## Description

### FIELD OF THE INVENTION

The invention relates to the design and expression of proteins with a symmetrical structures.

The invention relates to synthetic proteins with functional properties such as metal binding and enzymatic activity.

### BACKGROUND OF THE INVENTION

The functional and structural diversity of proteins has inspired researchers to engineer them for various applications. Recent examples have demonstrated the engineering of proteins as enhanced catalysts, vaccines, biosensors, and building blocks for 2D/3D frameworks [McConnell et al. ACS Synth. Biol. 2020, 9, 381-391; Brouwer et al. Cell 2021, 184, 1188-1200; Schuster Biosensors 2018, 8, 40; Chen et al. J. Am. Chem. Soc. 2019, 141, 8891-8895; Pyles et al. Nature 2019, 571, 251-256; Zhang et al. Nat. Commun. 2020, 11, 1-12; Ben-Sasson et al. Nature 2021, 589, 468-473]. In many cases, proteins with new functions are obtained via the redesign of existing proteins. Advances in computational protein design have stimulated the development of unique proteins with various conformations and functionality [Kuhlman & Bradley Nat. Rev. Mol. Cell Bio. 2019, 20, 681-697]. Therefore, protein engineers are not limited to re-purposing natural proteins and are able to expand their toolkit with new molecules with improved properties. Symmetric proteins are highly desirable due to their stability and versatility as building blocks for the development of 2D/3D assemblies [Yeates Annu. Rev. Biophys. 2017, 46, 23-42]. Recently, we designed an exceptionally stable, symmetric *β-*propeller protein called Pizza [Voet et al. Proc. Natl. Acad. Sci. U.S.A. 2014, 111, 15102-15107]. To showcase its functional potential, Pizza was redesigned to obtain protein assemblies, artificial enzymes, and high affinity scaffolds for various metals and metal-oxo clusters [Vrancken et al. J. Struct. Biol.: 4 2020, 100027; Clarke et al. Chem. Commun. 2019, 55, 8880-8883; Voet et al. Angew. Chem. Int. Ed. 2015, 127, 9995-9998; Vandebroek et al. Chem. Commun. 2020]. However, Pizza lacks an extensively modifiable interface which limits its capacity to bind more complex molecules.

### SUMMARY OF THE INVENTION

Advances in computational protein design have allowed for the development of new proteins with unique properties. Symmetric designer proteins have remarkable stability and can serve as versatile building blocks for the creation of macromolecular assemblies. In this work we describe the development of SAKe: A new symmetric, stable protein building block with modifiable loops. Following the observation of pH induced 3D self-assembly, we engineered metal binding sites along the protein's internal rotational axis to fabricate 2D surface arrays. Using atomic force microscopy, we demonstrated Cu(II) dependent on-surface 2D self-assembly. This work showcases a stable and highly modifiable SAKe protein scaffold, which holds promise as a building block for the creation of multi-functional macromolecular materials.

The invention is further summarised in the following statements. 1. A polypeptide comprising a sequence having at least 60% identity with NGRIY₅AVG₈G₉-Xₙ-LNSVE₁₄AY₁₆DP₁₈ETDEW₂₃SFVAPM₂₉TTPR₃₃SGVG₃₇VAV₄₀L [SEQ ID NO:32] or comprising 2 to 9 repeats of said sequence, wherein Xₙ are between 1 and 15 amino acids, wherein x can be any amino acid,
and wherein the amino acids Tyr₅, Gly₈, Glyg, Tyr₁₆, Trp₂₃, Arg₃₃, and Val₄₀ in said sequence are conserved.

The amino acids X can be any of the 20 natural amino acids encountered in polypeptides as well as modified versions thereof as obtained by post-translation modification. Other side chain can be envisaged when synthetic peptides are produced as ling as the amino acids can be incorporated via its NH₂ and COOH group in a polypeptide.

2. The polypeptide according to statement 1, wherein the amino acids Tyr₅, Gly₈, Gly₉, Glu₁₄, Tyr₁₆, Pro₁₈, Trp₂₃, Met₂₉, Arg₃₃, and Gly₃₇ and Val₄₀ in said sequence are conserved.

3. The polypeptide according to statement 1 or 2, comprising between 2 to 9 repeats of said sequence.

4. The polypeptide according to any one of statements 1 to 3, comprising 2, 3 or 6 repeats of said sequence.

5. The polypeptide according to any one of statements 1 to 4, wherein said sequence has at least 70, 80, 90 or 95% identity with SEQ ID NO:32 or is identical to SEQ ID NO:32.

6. The polypeptide according to any one of statements 1 to 5, wherein Xₙ are between 5 to 15 amino acids, or between 5 to 10 amino acids.

7. The polypeptide according to any one statements 1 to 6, wherein one or more of said repeats are separated from each other with 1 up to 5 amino acids.

8. The polypeptide according to any one of statements 1 to 7, wherein one or more of said repeats are immediately adjacent to each other.

9. The polypeptide according to any one of statements 1 to 8, wherein all of said repeats are immediately adjacent to each other.

10. The polypeptide according to any one of statements 1 to 9, wherein all repeats are immediately adjacent to each other.

11. The polypeptide according to any one of statements 1 to 10, wherein the repeats of said sequence are identical, with exception of the amino acids Xₙ.

12. The polypeptide according to any one of statements 1 to 10, comprising a first repeat and a second repeat of said sequence wherein said first and second repeat occur alternating in said polypeptide.

13. A polypeptide which is multimer of non-covalently bound polypeptides as defined in any one of statements 1 to 9.

14. The polypeptide according to claim 13, which is a hexamer of 3 non non-covalently bound polypeptides as defined in any one of claims 1 to 9 having two repeats.

15. The polypeptide according to claim 13, which is a hexamer of 2 non non-covalently bound polypeptides as defined in any one of claims 1 to 9 having 3 repeats. The invention further relates to nucleic acids encoding these polypeptides, as well as expressions vector comprising these nucleic acids, and bacterial yeast or eukaryotic cells comprising these vectors.

16. A method of producing a functional protein comprising the steps of:
a) providing a polypeptide according to any one of claims 1 to 13, wherein Xₙ is random selected or designed by an in silico method,
b) generating a multimeric protein of said polypeptides,
c) testing the multimeric protein for the function, and
d) selecting the multimer protein with the function.

17. The method according to claim 16, wherein the function is protein binding, an enzymatic activity, or the binding to an organic molecule.

18. The method according to claim 16 or 17, further comprising the step of determining whether the multimeric protein has rotational symmetry.

19. The method according to any one of claims 17 to 18, further comprising the step of determining whether the multimeric protein is stable at pH below 4, or wherein the multimeric protein is resistant against proteolytic degradation.

20. The method ACCORDING to any one of claims 17 to 19, further comprising determining whether said multimeric protein assembles into quaternary structures, such as fibres, tubes, three dimensional cages or two-dimensional layers.

### DETAILED DESCRIPTION

### Figure legends

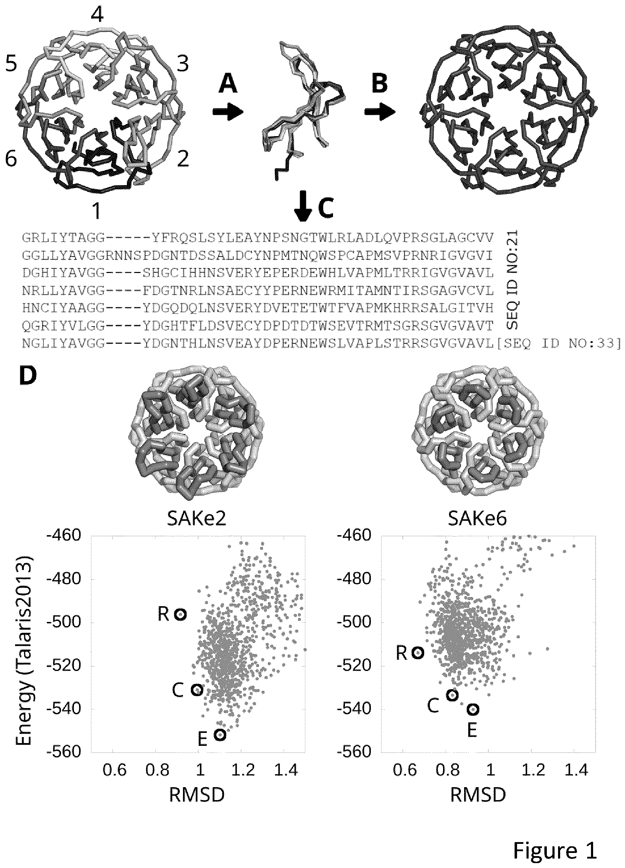

**Figure 1****.** Overview of the RE₃Volutionary design strategy. The human keapl *β-*propeller was used as a template (PDB: 1ZGK). **A.** The blades were separated. **B.** Using Rosetta SymDock, a perfect sixfold symmetric protein backbone was constructed from one blade. **C.** Simultaneously, a multiple sequence alignment (MSA) was constructed from the six unique blades. **D.** Putative ancestral sequences, derived from the MSA using the FastML server, are mapped on the perfect symmetric backbone. The resulting models were evaluated by their Rosetta Talaris2013 energy score and root mean square deviation (RMSD) from the ideal symmetric backbone. For each SAKe type, three designs were experimentally tested.

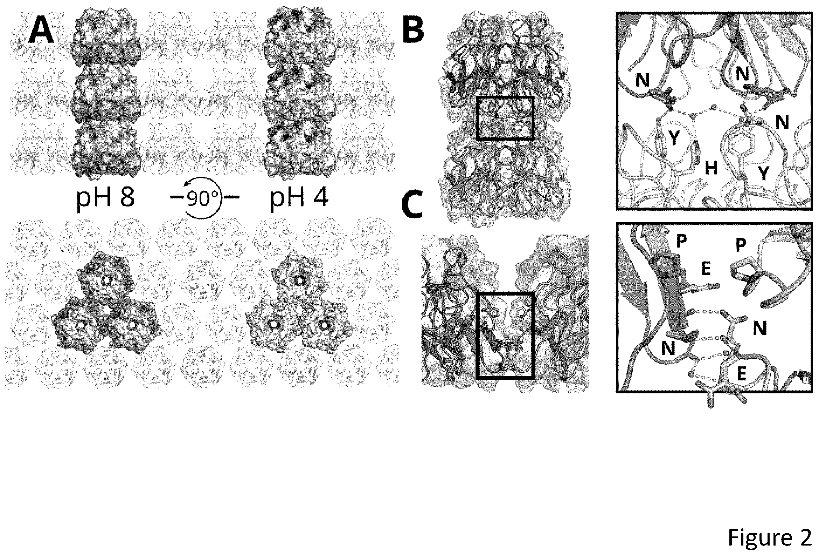

**Figure 2****. A.** APBS calculated surface electrostatics for the S6BE normal crystal structure at pH 8.0 and the self-assembled crystal structure at pH 4.0. **B.** Close-up of the axial hydrogen bonding networks found in the self-assembled S6BE crystal. **C.** Close-up of the lateral hydrogen bonding networks found in the self-assembled S6BE crystal. Additionally, there is a hydrophobic interaction between two prolines (5 °A distance). The only structural difference between the normal and self-assembled S6BE crystals is found in the alternative conformations of the lateral glutamates, here shown in slate/wheat (pH 4) and white (pH 7-8).

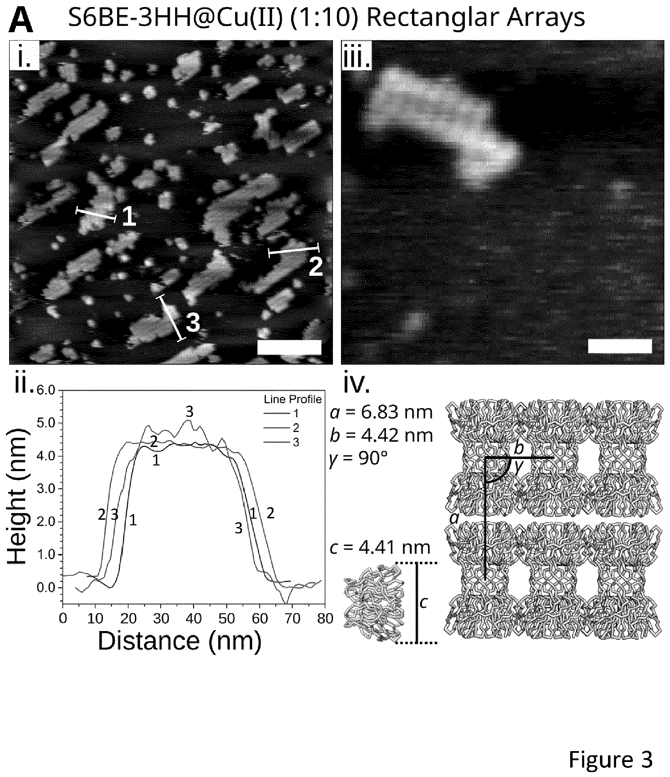

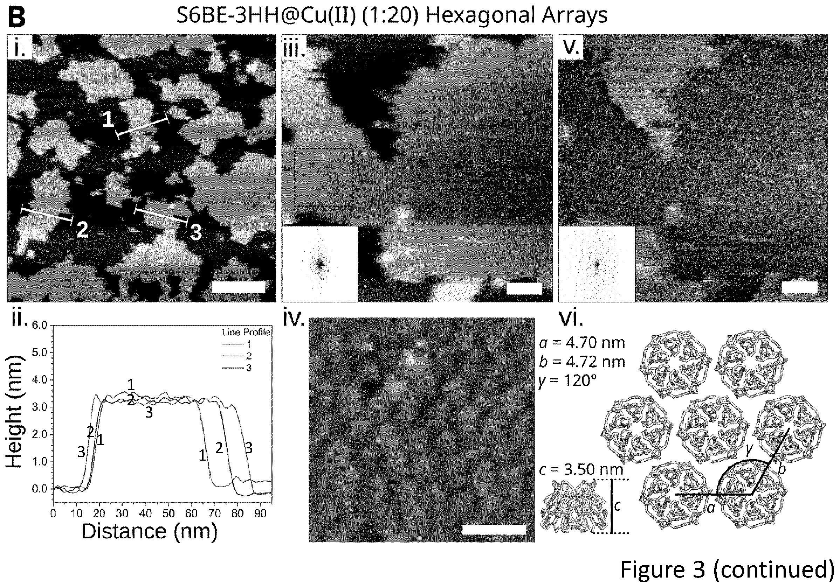

**Figure 3****.** AFM imaging of S6BE-3HH:Cu(II) 2D arrays. **A.** Rectangular-dimeric lattices are formed at a ratio of 1:10 (protein:Cu(II))**.(i)** Topography map of 1*µ*M S6BE-3HH, 10 *µ*M Cu(II) (100 nm scale bar) and **(ii)** selected line profiles. **(iii)** Topography map of 0.5 *µ*M S6BE-3HH, 5 *µ*M Cu(II) (20 nm scale bar). **(iv)** Dimensions calculated from the packing of a S6BE-3HH crystal (PDB code 7OPU)). **B.** Hexagonal lattices are generated at a ratio of 1:20 (1*µ*M S6BE-3HH:20*µ*M Cu(II)). **(i)** Topography map (100 nm scale bar) and **(ii)** selected line profiles. **(iii)** Topography map (20 nm scale bar), **(iv)** filtered zoom image of selected region (scale bar = 10 nm) and **(v)** phase map (20 nm scale bar). **(vi)** Dimensions calculated from the packing of a S6BE-3HH crystal (PDB code 7OPV)). All AFM imaging was performed in 20 mM MES buffer (pH 5.6) and on a mica surface.

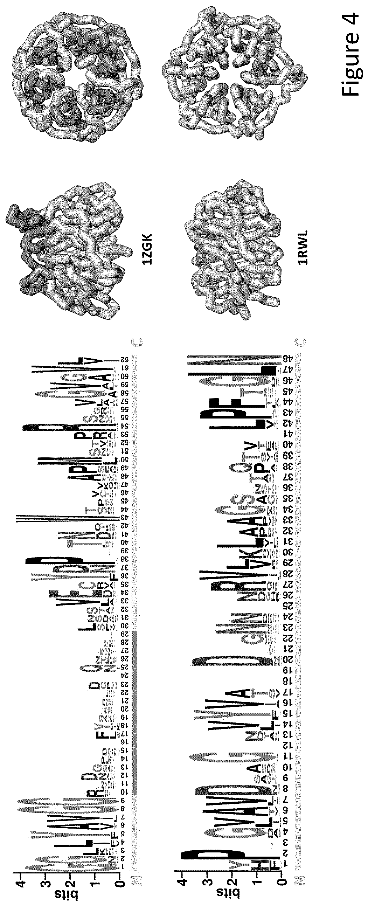

**Figure 4****:** Sequence logos generated with Consurf and WebLogo,1,2 using 150 sequences from the NR proteins database and the default Consurf settings. (Top) Logo generated from the second blade of the human keapl β-propeller (PDB code: 1ZGK).3 A clear region with low conservation corresponds to the protein's loops. (Bottom) Logo generated from the third blade of the Mycobacterium tuberculosis PknD β-propeller (PDB code: 1RWL).4 This protein was the template for the design of Pizza and lacks large variable regions.5

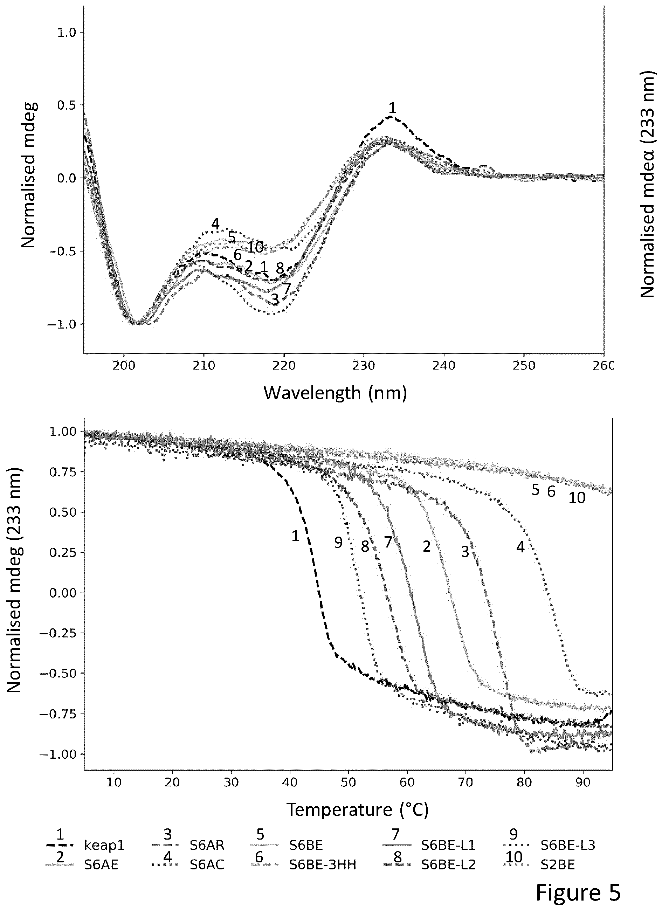

**Figure 5****:** (Left) Circular Dichroism (CD) spectra of our SAKe proteins were similar to that of the parent human keapl β-propeller. (Right) The CD signal at 233 nm was followed in function of temperature. Compared to keapl, all SAKe designs have an improved melting temperature. Between SAKe designs, loop length and composition seem to have the more pronounced impact on thermal stability. When loops are conserved (as is the case between S6A proteins), the scaffold sequence can still have a significant influence as well.

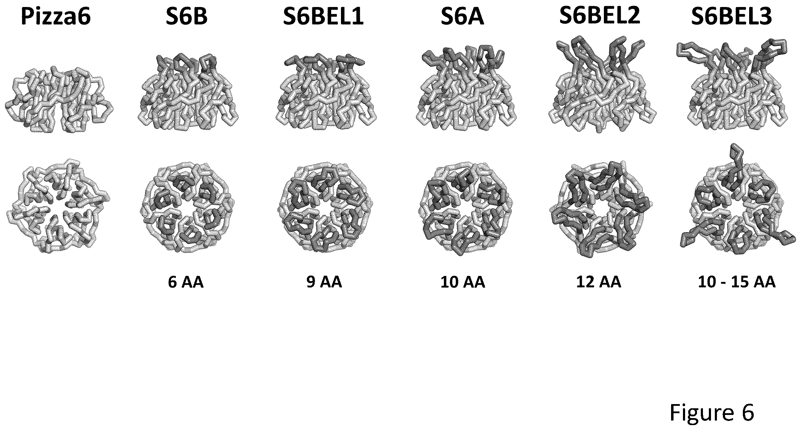

**Figure 6****:** Top and side views of crystal structures of Pizza6, A-type SAKe (S6A), B-type SAKe (S6B) , S6BE-L1, S6BE-L2 and S6BE-L3. The modular loops are colored in slate and their respective lengths are given. For S6BE-L2, the loops were too flexible and not visible in the electron density map. For visualization purposes, they have been modelled in this figure.

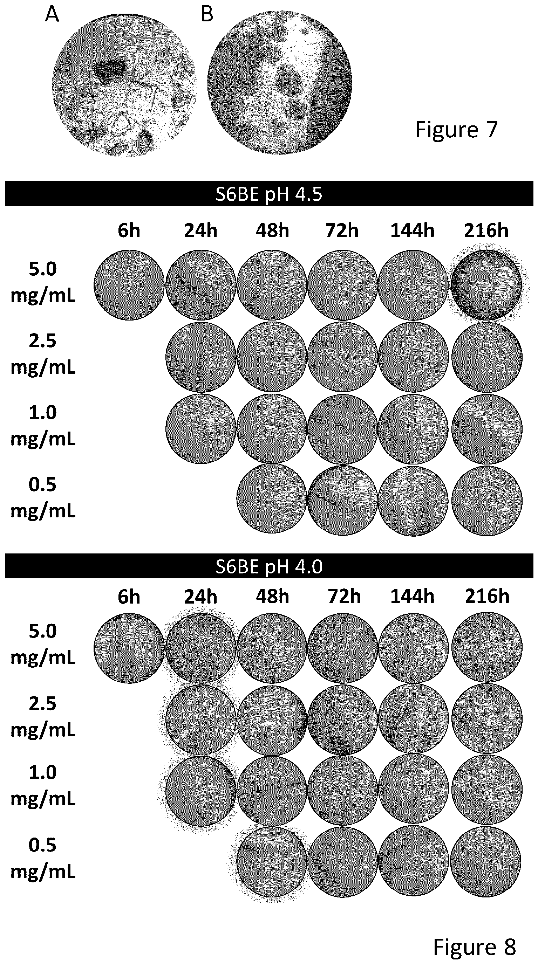

**Figure 7****:** Self assembled crystals of S6BE, grown in (A) 50 mM Na acetate - acetic acid pH 4.0 and (B) 50 mM Na citrate - citric acid pH 4.0. Both batches grew crystals after overnight dialysis of 20 mg/mL protein at 4 °C. The bubbles have an approximate diameter of 5 mm.
**Figure 8****:** Various concentrations of S6BE were dialyzed in parallel to either 50 mM Na citrate - citric acid pH 4.0 or 4.5. At pH 4.5, crystals only grew after 144h to 216h. At pH 4.0, the proteins readily crystallized after 6h to 72h, depending on their concentration. The first pictures that show crystals are marked in green. The bubbles have a radius of approximately 5 mm.

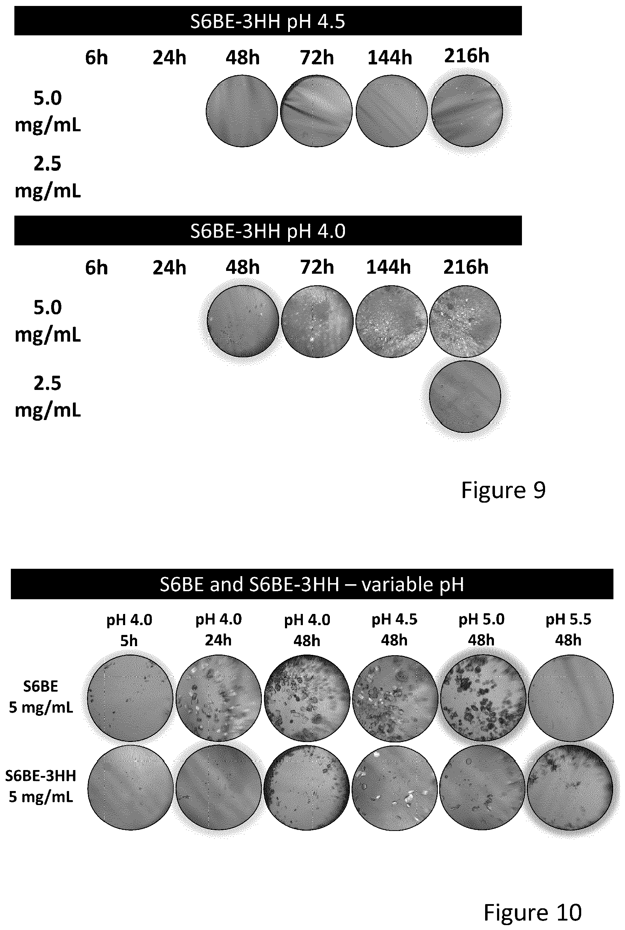

**Figure 9****:** Various concentrations of S6BE-3HH were dialyzed in parallel to either 50 mM Na citrate - citric acid pH 4.0 or 4.5. At pH 4.5, crystals only grew after 144h to 216h. At pH 4.0, the proteins readily crystallized after 24h to 216h, depending on their concentration. The first pictures that show crystals are marked in green. The bubbles have a radius of approximately 5 mm.
**Figure 10****:** 5 mg/mL aliquots of S6BE and S6BE-3HH were dialyzed to 50 mM Na citrate citric acid at varied pH. S6BE yielded more crystals and they grew faster than S6BE-3HH. At pH 4.5, crystals remained stable. From pH 5.0 onward they dissolved again. The first pictures that show crystals were marked in green, while the first notices of disassembly were marked in red. The bubbles have a radius of approximately 5 mm.

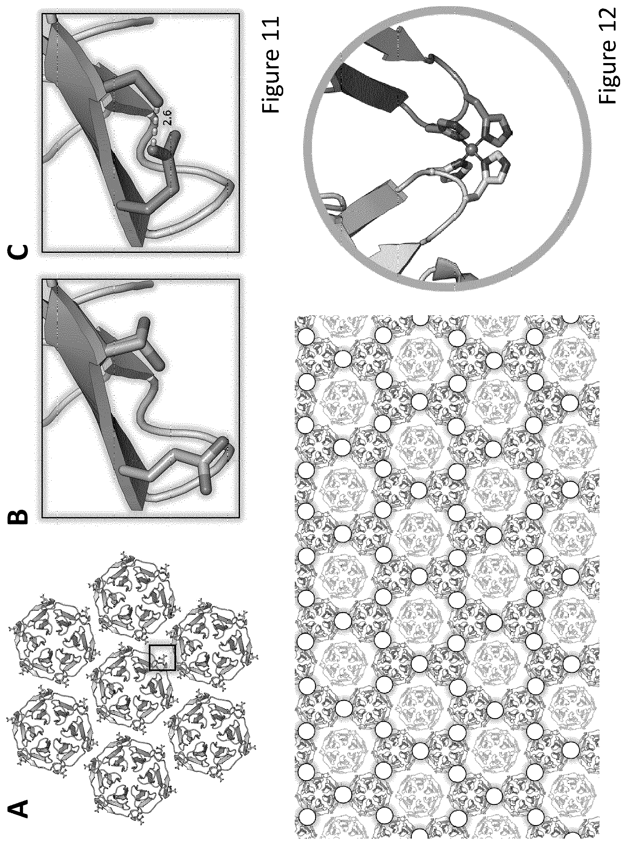

**Figure 11:** (A) The hexagonal crystal packings of normal (0.2 M K formate, 20% (w/v) PEG3350) and self-assembled (50 mM Na acetate - acetic acid pH 4.0) S6BE crystals are identical. The only obvious difference is found in the alternative rotamers for the lateral Glu. (B) In the normal crystal these Glu point outwards (0.48 occupancy of carboxy group, alternative rotamers were not visible). (C) The protonated Glu in the self-assembled crystal turn inward to form a putative hydrogen bridge with a serine hydroxyl group. Additionally, electrostatic repulsion in the normal crystal might direct the normal crystal's Glu outward.

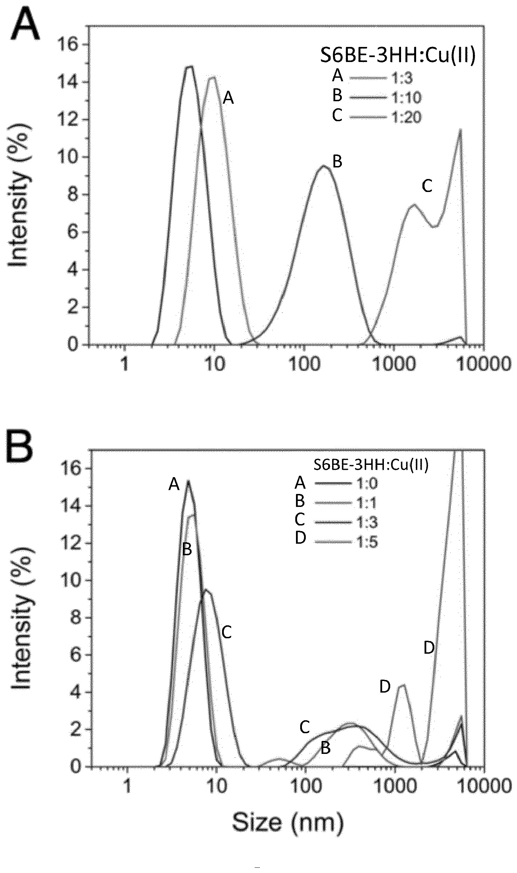

**Figure 12****:** To enable metal-induced self-assembly, three double-histidine sites were added in S6BE (creating S6BE-3HH). A closely packed structure resembling the self-assembled crystal structure is obtained through a combination of metal-mediated and complementary interactions of adjacent proteins. The vacancies of the hexagonal lattice are likely to be occupied by S6BE-3HH proteins.

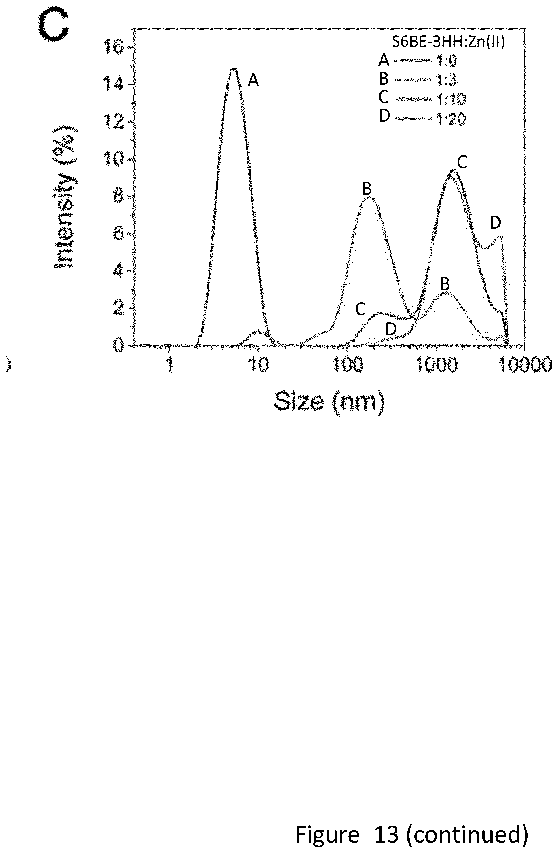

**Figure 13****:** DLS spectra of S6BE-3HH with different ratios of Cu(NO3)2 in MilliQ pH 7.0 (A) and 20 mM MES pH 5.6 (B). DLS spectrum of S6BE-3HH with different ratios of Zn(NO3)2 in 20 mM MES pH 5.6 (C).

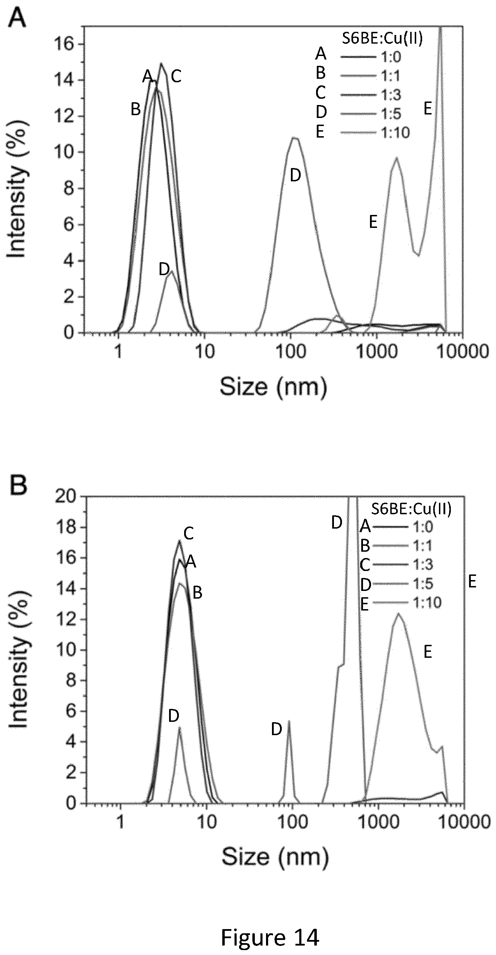

**Figure 14****:** DLS spectra of S6BE with different ratios of Cu(NO3)2 in MilliQ pH 7 (A) and 20 mM MES pH 5.6 (B).

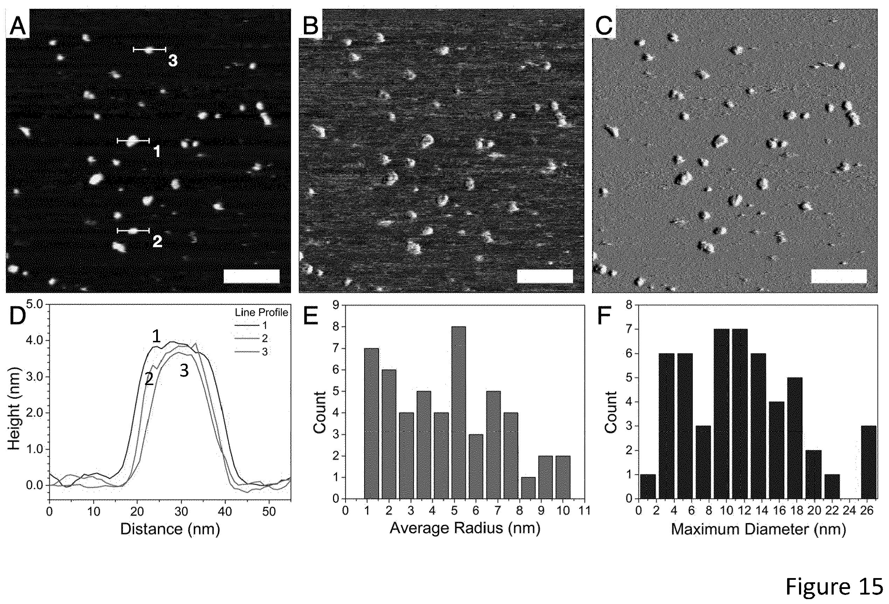

**Figure 15****:** AFM of 1 µM S6BE-3HH imaged in 20 mM MES pH 5.6 on mica. (A) Topography map, (B) phase map and (C) amplitude map. The scale bar on all images is 100 nm. (D) Height traces corresponding to the line profiles taken from the Topography map.
   Particle analysis of the topography, (E) average radius and (F) maximum diameter.

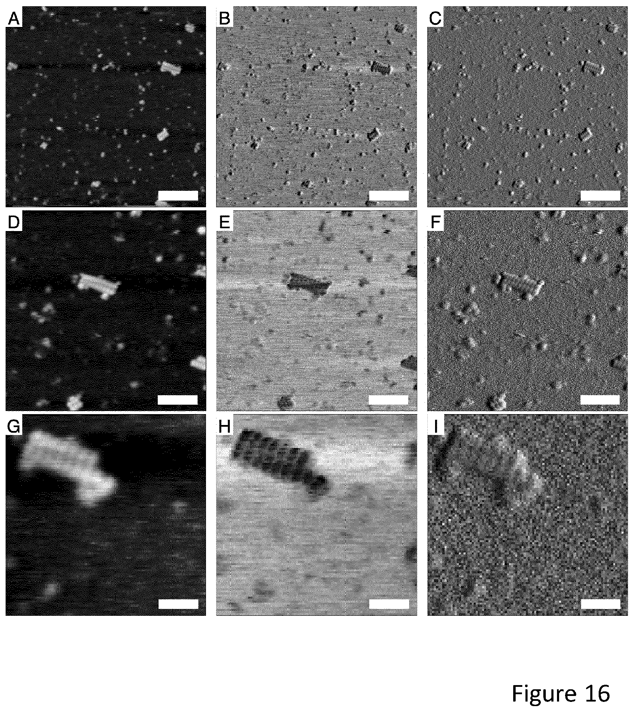

**Figure 16****:** AFM of 0.5 µM S6BE-3HH, 5 µM Cu(NO3)2 imaged in 20 mM MES pH 5.6 on mica. Topography maps (A, D & G), phase maps (B, E & H) and amplitude maps (C, F & I). The scale bar is 100 nm for images A-C, 50 nm for D-F and 20 nm for G-I.

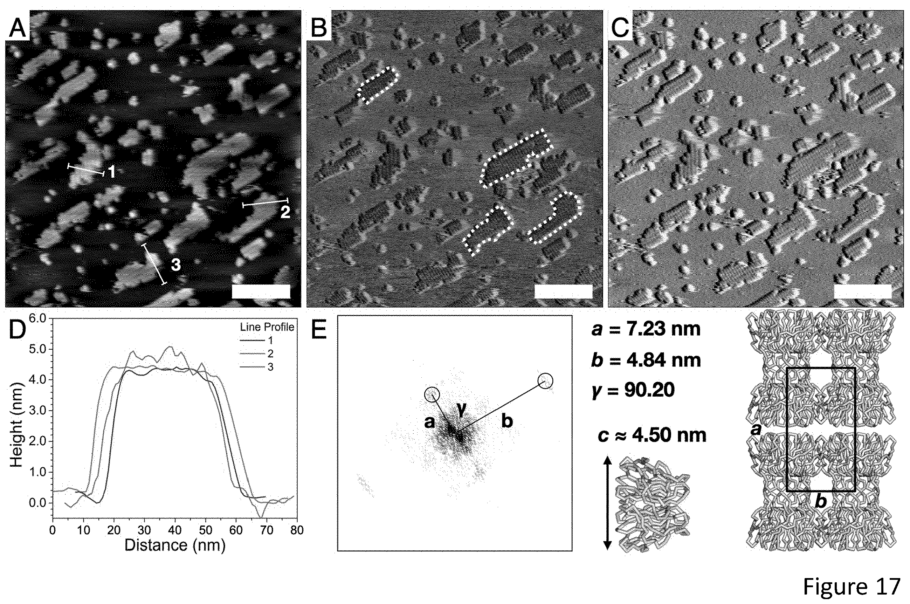

**Figure 17****:** AFM of 1.0 µM S6BE-3HH, 10 µM Cu(NO3)2 imaged in 20 mM MES pH 5.6 on mica. Topography map (A), phase map (B) and amplitude map (C). The scale bar is 100 nm. (D) Height traces corresponding to the line profiles taken from the topography map (A). (E) 2D-FFT map of the highlighted area selections in phase map (B), and schematic of the array's unit cell derived from the calculated distances of the 2D-FFT map and height traces (D).

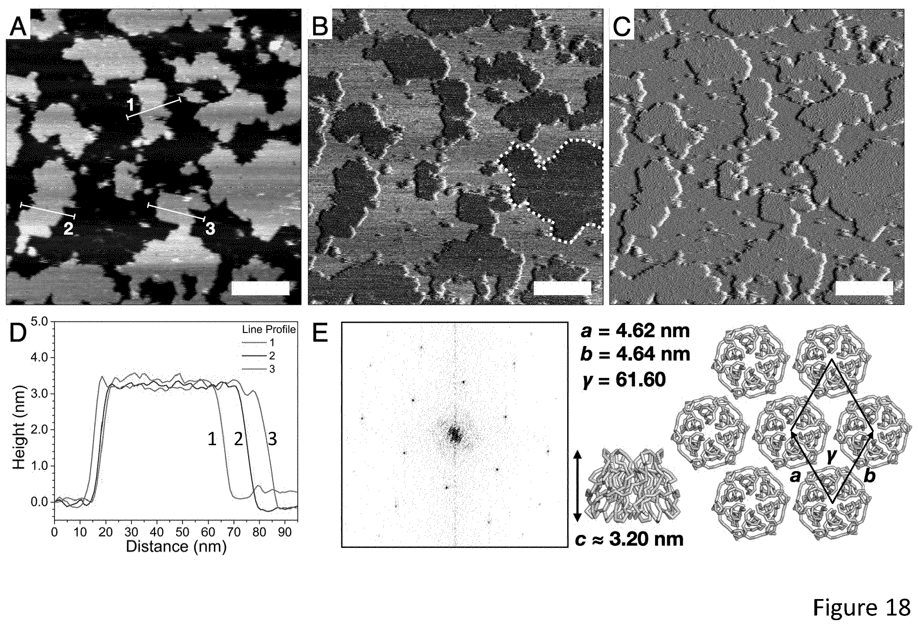

**Figure 18****:** AFM of 1.0 µM S6BE-3HH, 20 µM Cu(NO3)2 imaged in 20 mM MES pH 5.6 on mica. Topography map (A), phase map (B) and amplitude map (C). The scale bar is 100 nm. (D) Height traces corresponding to the line profiles taken from the topography map (A). (E) 2D-FFT map of the highlighted area selection in phase map (B), and schematic of the array's unit cell derived from the calculated distances of the 2D-FFT map and height traces (D).

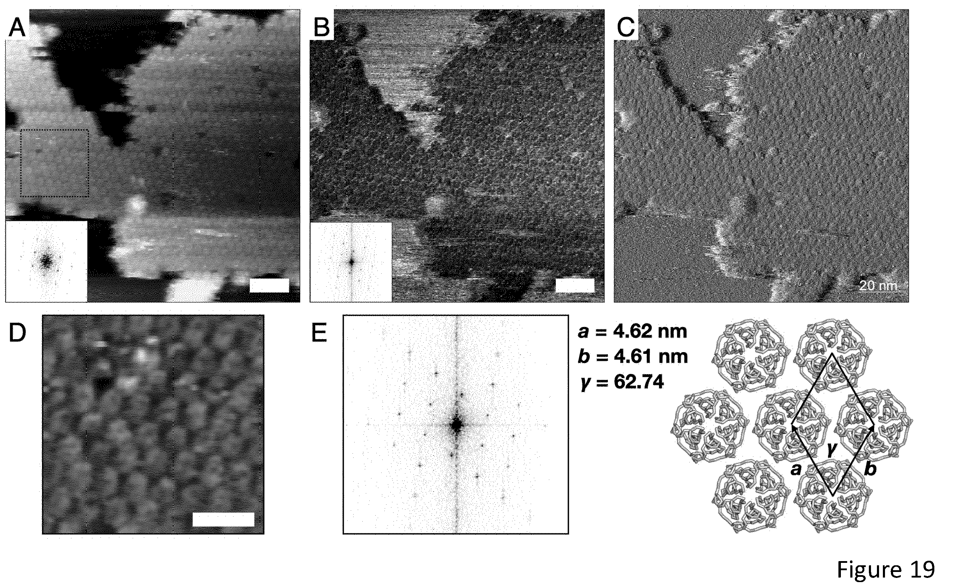

**Figure 19****:** AFM of 1.0 µM S6BE-3HH, 20 µM Cu(NO3)2 imaged in 20 mM MES pH 5.6 on mica. Topography map and 2D-FFT (A), phase map and 2D-FFT (B) and Amplitude map (C). The scale bar is 20 nm for A-C. (D) Projection and filtered image corresponding to the area selection in the topography map (A). The scale bar is 10 nm. (E) 2D-FFT map of the phase image (B), and schematic of the array's unit cell derived from the calculated distances of the 2D-FFT map.

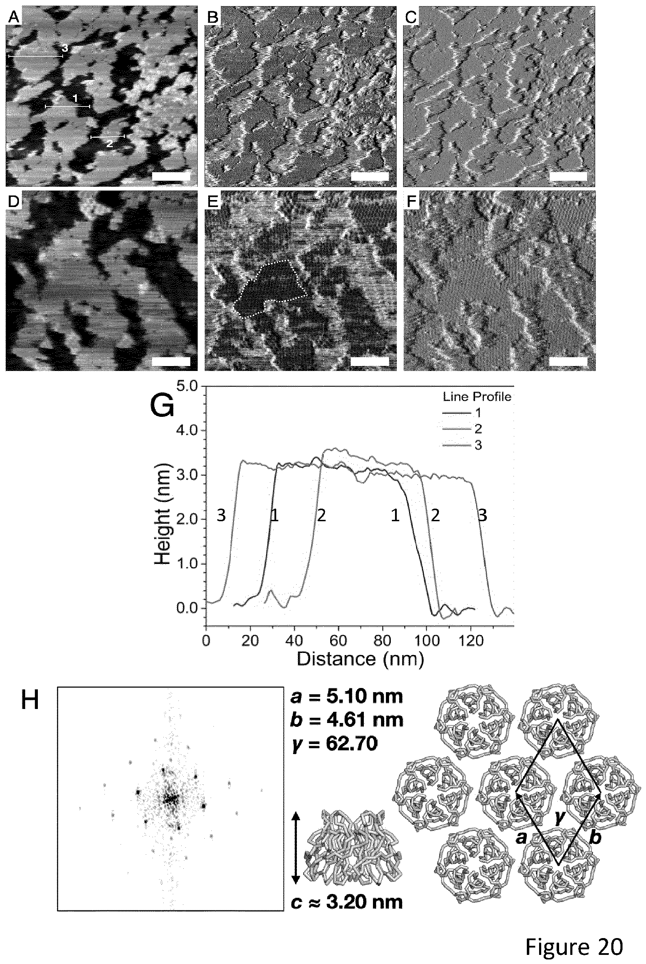

**Figure 20****:** AFM of 1.0 µM S6BE-3HH, 30 µM Cu(NO3)2 imaged in 20 mM MES pH 5.6 on mica. Topography maps (A & D), phase maps (B & E) and amplitude maps (C & F). The scale bar is 100 nm for A-C and 50 nm for D-F. (G) Height traces corresponding to the line profiles taken from the topography map (A). (E) 2D-FFT map of the highlighted area selections in phase map (E), and schematic of the array's unit cell derived from the calculated distances of the 2D-FFT map and height traces (G).

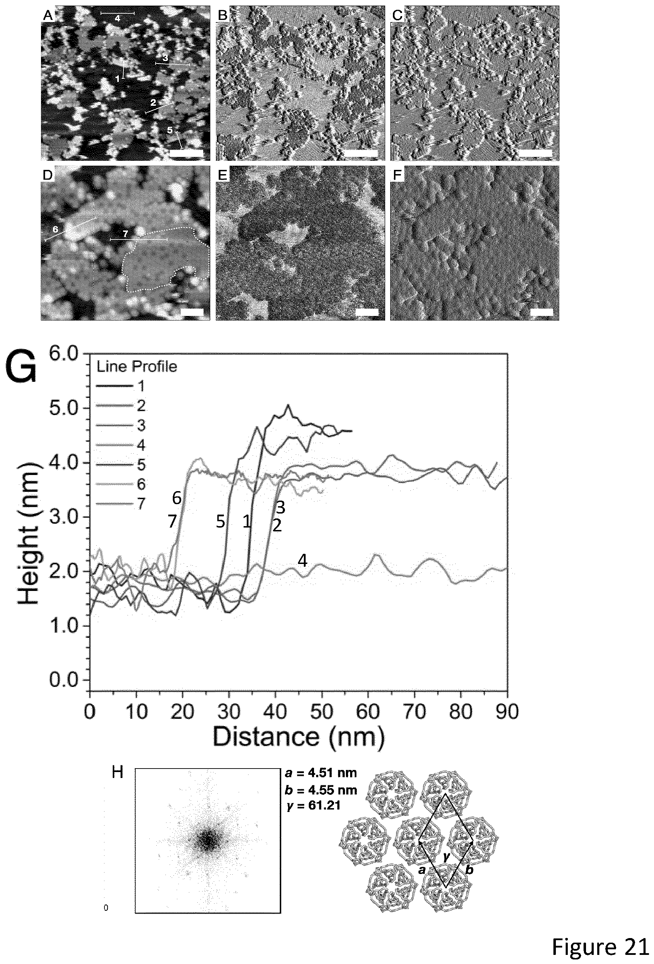

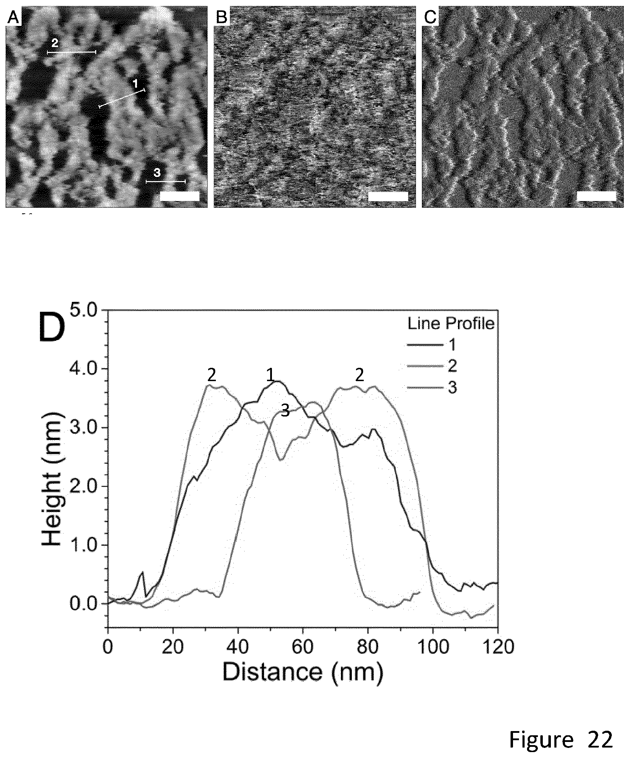

**Figure 21****:** AFM of 1.0 µM S6BE-3HH, 20 µM Cu(NO3)2 imaged in 20 mM MES pH 5.6 on HOPG. Topography maps (A & D), phase maps (B & E) and amplitude maps (C & F). The scale bar is 100 nm for A-C and 20 nm for D-F. (G) Height traces corresponding to the line profiles taken from the topography maps (A & D). (E) 2D-FFT map of the highlighted area selections in topography map (D), and schematic of the array's unit cell derived from the calculated distances of the 2D-FFT map. Figure 22: AFM of 1.0 µM S6BE-3HH, 10 µM Zn(NO3)2 imaged in 20 mM MES pH 5.6 on Mica. Topography map (A), phase map (B) and Amplitude map (C). The scale bar is 100 nm. (D) Height traces corresponding to the line profiles taken from the topography map (A).

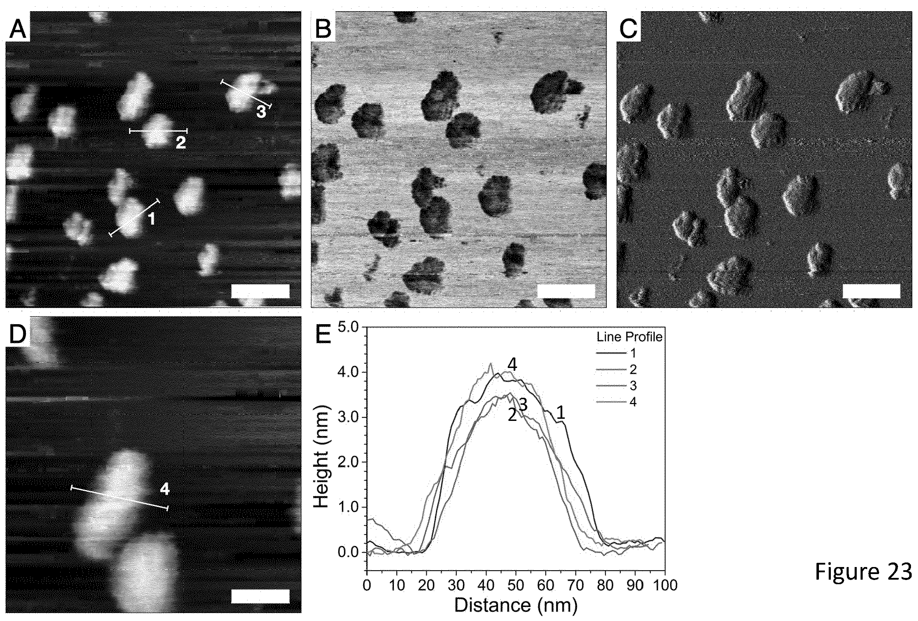

**Figure 23****:** AFM of 1.0 µM S6BE, 20 µM Cu(NO3)2 imaged in 20 mM MES pH 5.6 on mica. Topography maps (A & D), phase map (B) and amplitude map (C). The scale bar is 100 nm for A-C and 50 nm for D. (E) Height traces corresponding to the line profiles taken from the topography maps (A & D).

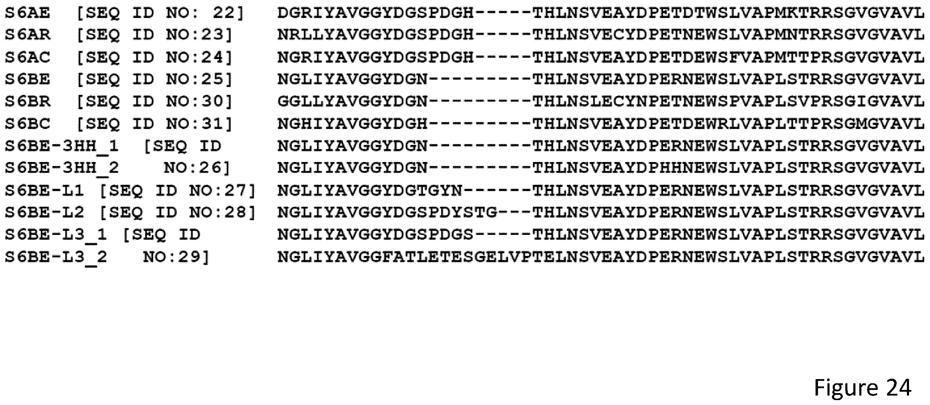

**Figure 24****:** Manual sequence alignment of all SAKe design sequences. Each entry contains only one blade. Proteins that have two blades per repeat span two entries. The amino acid sequences between the GG and LNS motifs are annotated as the protein's variable loop regions.

Herein, we successfully designed and engineered an improved protein building block named the Self-Assembling Kelch (SAKe) protein. SAKe has a stable, symmetric design with readily accessible loops that can be varied in both sequence and length to later bind larger molecules or scaffold a catalytic site. To demonstrate SAKe's versatility, we modified its structure to undergo metal-mediated self-assembly into 2D surface arrays. This highlights SAKe as a promising new protein scaffold which can be readily redesigned to target various applications.

Through an investigation into naturally occurring pseudosymmetric proteins, we identified the human keapl Kelch protein as a promising template for the development of SAKe [Beamer et al.. Acta. Crystallogr., Sect. D.: Biol. Crystallogr. 2005, 61, 1335-1342]. Kelch repeat proteins are *β*-propeller proteins composed of six nearly identical tandem sequence repeats that fold into 4-stranded anti-parallel sheets around a central cavity [Adams et al. Trends Cell Biol. 2000, 10, 17-24].This structural family has well-conserved blades, with the first and second strands connected by loops that vary in length and sequence. Using a computational procedure that combines ancestral sequence reconstruction with computational protein backbone optimization and subsequent sequence scoring, a new family of proteins named SAKe were derived from the keapl *β*-propeller (Figure 1) . Initially, six different SAKe proteins were designed and evaluated. They varied in their core amino acid sequence and length of surface loops. The variable "top-side" loops sit between the inner two *β*-strands of the propeller's blades; Atype SAKe (S6AE, S6AR and S6AC) loops have 10 amino acids while B-type SAKe (S6BE, S6BR and S6BC) loops have 6. Four of the six proteins were successfully expressed and readily crystallized: three A-type SAKes (S6AE, S6AR and S6AC) and one B-type SAKe (S6BE). Each protein has perfect 6-fold symmetry, with a central cavity surrounded by flexible loops on the "top" face. Circular Dichroism (CD) spectroscopy at elevated temperatures highlights their exceptional thermal stability, whereby S6BE exhibits a melting temperature (*Tₘ*) of over 95 °C.

To investigate loop modification and further functionalisation, we created three loop variants of S6BE (Figure 6). S6BE-L1 was created by conserving the intersection of S6Atype and S6B-type loops, while adding in 4 amino acids. For S6BE-L2, a larger part of the S6A-type loop was conserved with the addition of 4 amino acids. These 4 amino acid sequences were random Tyr-containing combinations of frequently occurring residues in nanobody CDR motifs [Zuo et al. BMC genomics 2017, 18, 797], and are not found in the natural Kelch proteins. We also created S6BE-L3, a 3-fold symmetric variant with two long loops of various length through grafting them from the Kelch domain of human KBTBD5 [Canning et al. Journal of Biological Chemistry 2013, 288, 7803-7814]. All 3 proteins were successfully purified, and their structures were confirmed via X-ray diffraction (XRD). However, the S6BE loop variants were found to be less stable than the original S6BE, with *Tₘ* dropping to a minimum of 51.7 °C as the loop lengths increased (Figure 5). Nonetheless, all of these designs are significantly more stable than the template keapl *β*-propeller, which unfolds at 44.1 °C.

SAKe's symmetry and modifiable interfaces make it an ideal building block for construction of macromolecular assemblies. Interestingly, while dialyzing S6BE to low pH we observed pH induced self-assembly (Figure 7. This pH responsive behaviour was further investigated through dialysis experiments and XRD. The tipping point for this self-assembly was found to be around pH 4.0 (Figure 8 and 9). At pH 4.0, large crystals were observed for S6BE within a few hours up to two days, depending on the concentration. In contrast, crystals only formed after 6 days at 5 mg/mL when dialyzing to pH 4.5. The self-assembled crystals grown at pH 4.0 remained stable at pH 4.5. However, they disassembled at pH 5.0 (Figure 10), but readily re-assembled and formed crystals again when the pH was dropped to pH 4.0. To understand this reversible self-assembly mechanism, we investigated the crystals formed at different pH with XRD. These self-assembled structures revealed identical packing to the crystals grown via vapor diffusion at pH 7.0-8.5. At pH 8.0, His (pKa3 6.08-6.90) and Glu (pKa 4.3-4.4) are deprotonated and the net positive charge of S6BE likely causes mutual repulsion. However, at pH 4 these residues are protonated. Due to its symmetry and dipole-like character, S6BE shape and charge complementarity is then expected to allow for self-assembly into a tight hexagonal packing (Figures 2 and 11). A series of hydrogen bonds and hydrophobic interactions can then further stabilize the assembly. Similar mechanisms have been reported for the capsid proteins of cowpea chlorotic mosaic virus (CCMV) [Speir et al. Structure 1995, 3, 63-78; Lavelle et al. J. Phys. Chem. B 2009, 113, 3813-3819; Van Eldijk et al. J. Am. Chem. Soc. 2012, 134, 18506-18509].

The hexagonal packing and complementary interactions found in S6BE's self-assembled structures provide a promising starting point for its application as a supramolecular building block. Therefore, we attempted to rationally reengineer this protein to coordinate divalent metal-ions, and induce self-assembly into on-surface 2D arrays (Figure 11). We rationally selected 6 residues in S6BE to be mutated to His residues, creating the S6BE-3HH variant. These double His sites can be found on the bottom protrusions of the 1st, 3th and 5th blade, forming 3-fold rotational symmetry. Previous reports have combined a similar metal coordination strategy with naturally occurring symmetric protein oligomers to fabricate nano-particles and crystalline protein arrays [Salgado et al. Acc. Chem. Res. 2010, 43, 661-672; Huard et al. Nat. Chem. Biol. 2013, 9, 169-176; Suzuki et al. Nature 2016, 533, 369-373].

The metal-induced assembly of S6BE and S6BE-3HH proteins was first screened using Dynamic Light Scattering (DLS) in solution. Divalent metals (Cu(NO₃)₂ and Zn(NO₃)₂) were titrated into the protein solution in different buffers and pH, resulting in larger structures being formed as the ratio of metal:protein was increased (Figures 13 and 14). Interestingly, in both neutral and acidic conditions and at a ratio of 3:1 Cu(II):S6BE-3HH, an intermediate peak with a diameter of ≈ 10 nm can be recognized before the structures reached larger indistinguishable sizes at higher ratios.

To investigate the formation of on-surface 2D protein arrays, we utilized in solution amplitude-modulated atomic force microscopy (AFM) (Figure 3). In the absence of metals and on a muscovite mica surface, it was recognized that the optimal conditions for imaging S6BE and S6BE-3HH was in a mildly acidic buffer (20 mM MES pH 5.6). In these conditions, both proteins adhered to the surface and adopted monomeric species with some small aggregates (Figures 15). It is likely that in acidic conditions the proteins carry enough positively charged residues to form complementary interactions with the negatively charged mica surface. Organized 2D surface arrays were first observed for the S6BE-3HH protein at a ratio of 10:1 Cu(II):protein with an overall protein concentration of 0.5-1.0 µM, whilst imaging in 20 mM MES buffer (pH 5.6). The stable 2D surface crystals adopted high aspect ratio rectangular geometries with a preferred directional growth (Figure 3A). Through analysing the dimensions derived from the 2D-FFT of selected surface crystals and their height profiles, the unit cell of these arrays could be obtained with c ≈ 4.5 nm, a = 7.23 nm, b = 4.84 nm and γ = 90.20 (Figure 17).These unit cell dimensions correspond to arrays composed of S6BE-3HH dimers, and are similar to the 2D protein arrangements found in a S6BE-3HH crystal structure (Figure 3A(iv)). Therefore, it is highly likely that these arrays are composed of protein dimers arranged bottombottom, which are bridged via Cu(II) ions and complementary interactions. At a ratio of ≥ 20:1 (Cu(II):S6BE-3HH), the surface arrays were found to transition from the rectangular-dimer species to hexagonal-honeycomb lattice. The 2D-FFT of these crystals confirmed their highly crystalline hexagonal packing with unit cell dimensions of a = 4.62 nm, b = 4.64 nm and γ = 61.60 (Figures 3B and S18). This unit cell coupled with the height traces of individual domains (c ≈ 3.2 nm) suggested that these 2D surface arrays were similar in geometry to the planar protein packing in a S6BE-3HH crystal (a = 4.70 nm, b = 4.72 nm, *α* = 90.00, β = 90.00 and γ = 120.00). Similar crystalline structures were also observed on a highly oriented pyrolytic graphite (HOPG) surface (Figure 21). The unit cell of the hexagonal array domains derived from the 2D-FFT (a = 4.55 nm, b = 4.51 nm and *α* = 61.21) had similar dimensions to that obtained for the arrays on mica in the same conditions. However, the hexagonal arrays appear to have more vacancies and imperfections within the lattices. Through replacing Cu(NO₃)₂ with Zn(NO₃)₂, which is attributed with a similar atomic radius but a different tetrahedral coordination, no ordered arrays for S6BE-3HH could be obtained in the same imaging conditions (mica, 20 mM MES pH 5.6). Instead, aggregated proteins were observed with no obvious crystallinity (Figure 22). This highlights the importance of Cu(II) and its square-planar coordination in the formation of these arrays.

We also studied the assembly of S6BE in the presence of Cu(II), which does not contain His mutations on its bottom loops. At a ratio of 20:1 Cu(II):S6BE, again we observed amorphous aggregated proteins with no obvious crystallinity (Figure 23). This indicates the importance of the His mutations in S6BE-3HH and their role in facilitating Cu(II) coordination and subsequent assembly of 2D arrays. At pH 5.6, these His residues are close to their pKa and are likely to adopt a mixture of protonated and deprotonated states. The pKa may also be lowered and mediated by nearby local bases such as Glu, further facilitating deprotonation and assembly in the presence of excess of Cu(II). In addition, pH 5.6 is close to S6BE-3HH pI (5.32), where the proteins will be predominantly neutralized and will no longer repel each other. A similar mechanism has been previously reported for Zn-mediated protein assemblies at pH 5.5 [Brodin et al. Nat. Chem. 2012, 4, 375-382]. Though demonstrating the Cu(II) dependent structural self-assembly, we can suggest that the square planar coordination of Cu(II) combined with the bi-chelate His mutations and shape complementarity of S6BE-3HH are essential for connecting adjacent proteins into close packed 2D arrays (Figure 12).

Using a computational approach combining consensus design and Rosetta energy scoring, we successfully engineered SAKe: a new symmetric, stable protein scaffold with modifiable loops. We demonstrated that the loops can be varied in both length and sequence, highlighting their potential to be further optimized for the binding of clinically relevant molecules or programming of catalytic activity. Following SAKe's modification with metal binding sites, we observed Cu(II) induced self-assembly of on-surface 2D arrays. This work showcases SAKe as a highly modifiable protein scaffold which can double as a building block for the fabrication of 2D protein assemblies. In summary, SAKe's versatility holds great promise for the creation of high-impact biotherapeutics and innovative on-surface materials.

The following crystal structures were submitted to RCSB PDB: SAKe6AE (7ON6), SAKe6AR (7ON8), SAKe6AC (7ONA), SAKe6BE (7ONC and 7ONE), SAKe6BE3HH (7OP4, 7OPU and 7OPV), SAKe6BE-L1 (7ONG), SAKe6BE-L1 (7ON7) and SAKe6BE-L1 (7ONH).

### Overview of DNA and protein sequences:

SEQ ID NO:1 to SEQ ID NO: 19 (odd numbers): DNA sequences of designed SAKe proteins. The outermost emphasized 5'and 3'sequences contain a start codon, NdeI restriction site, stop codon and XhoI restriction site.

SEQ ID NO:1 to SEQ ID NO: 19 (even numbers): corresponding amino acid sequences.
S6AE [SEQ ID NO:1] 
GGGAATTCCATATGGATGGACGTATATACGCGGTTGGCGGGTATGATGGAAGTCCCGACGGTCATACGCA
CCTGAATTCTGTCGAAGCCTACGATCCCGAGACCGACACTTGGAGTCTGGTTGCCCCCATGAAAACTCGC
CGTTCTGGTGTAGGAGTTGCGGTCCTGGACGGCAGAATCTATGCCGTTGGCGGGTATGATGGATCCCCTG
ACGGCCACACTCACCTTAATTCAGTGGAGGCCTATGATCCCGAAACCGATACCTGGAGCTTGGTGGCCCC
CATGAAGACACGGCGGTCGGGCGTCGGCGTAGCGGTCTTGGATGGGAGAATATATGCAGTTGGTGGGTAC
GATGGGTCGCCTGATGGACACACTCATTTGAACTCCGTAGAAGCTTACGATCCGGAGACGGATACATGGT
CTCTTGTGGCACCTATGAAAACCCGGAGATCTGGCGTCGGAGTCGCCGTATTGGATGGCAGAATATACGC
TGTCGGGGGCTACGACGGCAGCCCTGACGGTCACACTCACCTTAATTCTGTAGAGGCATACGACCCCGAA
ACAGACACGTGGAGCCTGGTTGCTCCAATGAAAACACGCAGATCCGGCGTGGGGGTAGCGGTGCTGGATG
GCCGTATCTACGCGGTTGGAGGGTATGACGGCAGCCCCGATGGTCACACCCACCTGAATAGTGTGGAAGC
GTATGATCCTGAGACGGACACCTGGTCTTTGGTGGCTCCTATGAAAACGAGACGTTCAGGAGTAGGAGTT
GCAGTTTTGGACGGTCGTATTTATGCAGTTGGGGGATATGACGGATCCCCGGACGGCCACACGCACTTGA
ATTCAGTTGAAGCTTATGACCCAGAGACTGATACCTGGTCGCTGGTTGCTCCGATGAAAACGCGGCGGAA
CGGTGTTGGCGTGGCTGTCCTTTAACTCGAGCGG
S6AE [SEQ ID NO:2] 
MDGRIYAVGGYDGSPDGHTHLNSVEAYDPETDTWSLVAPMKTRRSGVGVAVL
 DGRIYAVGGYDGSPDGHTHLNSVEAYDPETDTWSLVAPMKTRRSGVGVAVL
 DGRIYAVGGYDGSPDGHTHLNSVEAYDPETDTWSLVAPMKTRRSGVGVAVL
 DGRIYAVGGYDGSPDGHTHLNSVEAYDPETDTWSLVAPMKTRRSGVGVAVL
 DGRIYAVGGYDGSPDGHTHLNSVEAYDPETDTWSLVAPMKTRRSGVGVAVL
 DGRIYAVGGYDGSPDGHTHLNSVEAYDPETDTWSLVAPMKTRRNGVGVAVL
S6AR [SEQ ID NO:3] 
GGGAATTCCATATGAATAGATTGTTGTACGCTGTTGGGGGGTATGATGGTAGTCCAGATGGCCATACCCA
CCTGAACTCCGTTGAGTGCTACGATCCAGAGACCAACGAATGGTCGCTTGTAGCACCTATGAATACCCGC
CGTTCAGGCGTTGGGGTAGCTGTTTTAAATCGCCTGCTGTACGCGGTTGGCGGGTACGACGGATCCCCTG
ATGGGCACACCCACTTGAACTCTGTAGAATGCTATGACCCTGAAACGAATGAGTGGTCCCTTGTCGCTCC
CATGAATACGAGACGTTCAGGAGTTGGAGTTGCAGTTTTAAACCGCCTTCTTTATGCGGTTGGCGGTTAC
GACGGTAGCCCAGATGGACACACTCACTTGAATTCTGTGGAGTGCTACGACCCTGAGACGAACGAATGGA
GTTTAGTTGCACCGATGAATACGCGCCGGAGCGGTGTAGGTGTAGCCGTGCTTAATCGGTTACTTTACGC
AGTAGGGGGTTACGACGGATCTCCTGACGGTCACACCCACTTAAACTCTGTCGAATGTTACGATCCTGAG
ACTAACGAGTGGTCTTTAGTCGCTCCCATGAATACCCGCCGCAGCGGCGTAGGCGTAGCGGTACTTAACC
GGTTGTTGTATGCAGTTGGCGGGTACGACGGGTCACCGGACGGCCACACGCACCTGAACTCTGTCGAATG
TTACGACCCCGAAACAAATGAGTGGAGCCTGGTGGCCCCTATGAACACTCGCAGATCAGGAGTCGGGGTA
GCGGTGTTAAACAGACTTCTGTATGCTGTGGGAGGCTACGATGGATCCCCGGACGGGCATACCCATCTGA
ATTCAGTGGAGTGTTATGATCCCGAGACGAACGAATGGTCGCTTGTAGCGCCCATGAATACGCGGAGAAG
CGGTGTCGGCGTTGCGGTCCTTTAACTCGAGCGG
S6AR [SEQ ID NO:4] 
MNRLLYAVGGYDGSPDGHTHLNSVECYDPETNEWSLVAPMNTRRSGVGVAVL
NRLLYAVGGYDGSPDGHTHLNSVECYDPETNEWSLVAPMNTRRSGVGVAVL
NRLLYAVGGYDGSPDGHTHLNSVECYDPETNEWSLVAPMNTRRSGVGVAVL
NRLLYAVGGYDGSPDGHTHLNSVECYDPETNEWSLVAPMNTRRSGVGVAVL
NRLLYAVGGYDGSPDGHTHLNSVECYDPETNEWSLVAPMNTRRSGVGVAVL
NRLLYAVGGYDGSPDGHTHLNSVECYDPETNEWSLVAPMNTRRSGVGVAVL
S6AC [SEQ ID NO:5] 
GGGAATTCCATATGAATGGCCGCATTTATGCCGTAGGGGGGTATGACGGATCCCCAGACGGACATACACA
TTTAAATTCGGTAGAGGCATACGACCCGGAAACGGACGAGTGGTCGTTTGTAGCTCCCATGACAACGCCA
CGGTCCGGAGTCGGGGTTGCCGTGCTTAATGGCCGTATATATGCCGTCGGGGGTTACGACGGATCGCCAG
ATGGGCACACTCATTTGAATAGTGTTGAAGCTTACGATCCGGAGACGGATGAGTGGTCATTTGTTGCTCC
GATGACAACCCCGCGTAGTGGTGTTGGAGTTGCAGTCCTGAACGGCCGGATCTATGCCGTCGGCGGTTAT
GACGGTTCTCCTGATGGACACACGCACTTGAATTCAGTAGAGGCTTACGACCCTGAAACAGATGAATGGT
CCTTTGTTGCCCCGATGACTACACCAAGATCGGGAGTTGGGGTGGCTGTCCTGAATGGGAGAATTTATGC
GGTAGGCGGATACGATGGTAGTCCAGATGGCCACACACACTTAAACTCCGTCGAGGCATACGACCCGGAG
ACGGATGAATGGTCATTCGTTGCACCAATGACTACACCTCGGTCCGGGGTCGGCGTGGCAGTATTAAACG
GGCGTATCTATGCCGTTGGTGGCTACGACGGAAGTCCCGACGGCCATACGCATCTGAACTCGGTGGAAGC
CTATGACCCCGAAACTGATGAATGGAGTTTTGTGGCGCCCATGACGACGCCTCGCTCCGGCGTCGGGGTA
GCTGTGCTGAACGGGCGGATTTACGCGGTTGGTGGTTATGATGGATCCCCTGACGGACATACTCACTTGA
ATTCTGTGGAAGCTTATGACCCAGAGACCGACGAGTGGTCTTTCGTGGCGCCGATGACGACACCCCGCTC
AGGAGTCGGAGTGGCGGTATTGTAACTCGAGCGG
S6AC [SEQ ID NO:6] 
MNGRIYAVGGYDGSPDGHTHLNSVEAYDPETDEWSFVAPMTTPRSGVGVA
VLNGRIYAVGGYDGSPDGHTHLNSVEAYDPETDEWSFVAPMTTPRSGVGV
AVLNGRIYAVGGYDGSPDGHTHLNSVEAYDPETDEWSFVAPMTTPRSGVG
VAVLNGRIYAVGGYDGSPDGHTHLNSVEAYDPETDEWSFVAPMTTPRSGV
GVAVLNGRIYAVGGYDGSPDGHTHLNSVEAYDPETDEWSFVAPMTTPRSG
VGVAVLNGRIYAVGGYDGSPDGHTHLNSVEAYDPETDEWSFVAPMTTPRS
GVGVAVL
S6BE [SEQ ID NO:7] 
GGGAATTCCATATGAATGGACTGATATACGCGGTTGGCGGATATGATGGAAACACGCATTTAAACTCTGT
TGAAGCGTATGATCCCGAACGCAATGAGTGGTCCTTAGTTGCACCACTTTCGACTCGGCGGTCGGGGGTA
GGGGTTGCAGTATTAAATGGGCTTATTTATGCAGTTGGAGGCTATGATGGAAATACACATCTGAATTCTG
TGGAGGCCTATGATCCCGAGCGGAATGAGTGGTCTCTGGTGGCCCCGCTGAGCACTCGCCGTTCAGGGGT
CGGCGTGGCCGTTCTGAACGGGCTTATATATGCCGTGGGGGGTTACGACGGCAACACACACTTAAATTCT
GTAGAAGCTTACGATCCCGAGAGAAACGAGTGGTCCCTTGTCGCTCCTTTATCCACCCGCAGAAGCGGTG
TGGGTGTGGCTGTATTAAACGGTCTGATTTATGCCGTAGGGGGATACGACGGTAATACACACCTGAATAG
CGTTGAGGCCTATGATCCGGAGCGCAACGAATGGTCGTTGGTTGCGCCTTTGTCAACTCGCCGGTCTGGT
GTGGGGGTAGCCGTGTTGAACGGCCTGATATATGCGGTGGGTGGGTATGATGGAAACACTCACCTGAACT
CGGTAGAGGCGTACGATCCAGAGCGCAACGAGTGGTCTCTGGTCGCCCCACTGTCGACTCGCCGGTCAGG
CGTTGGGGTTGCCGTACTTAACGGCCTGATTTATGCGGTCGGTGGTTATGACGGGAACACTCACCTGAAT
TCCGTGGAAGCTTATGACCCAGAACGCAATGAATGGAGTCTGGTTGCACCCCTGTCTACTCGTAGATCGG
GGGTCGGGGTAGCTGTCTTGTAACTCGAGCGG
S6BE [SEQ ID NO:8] 
MNGLIYAVGGYDGNTHLNSVEAYDPERNEWSLVAPLSTRRSGVGVAVL
NGLIYAVGGYDGNTHLNSVEAYDPERNEWSLVAPLSTRRSGVGVAVL
NGLIYAVGGYDGNTHLNSVEAYDPERNEWSLVAPLSTRRSGVGVAVL
NGLIYAVGGYDGNTHLNSVEAYDPERNEWSLVAPLSTRRSGVGVAVL
NGLIYAVGGYDGNTHLNSVEAYDPERNEWSLVAPLSTRRSGVGVAVL
NGLIYAVGGYDGNTHLNSVEAYDPERNEWSLVAPLSTRRSGVGVAVL
S6BE-3HH [SEQ ID NO:9] 
CCAATTCATATGTTAATTTACGCTGTGGGCGGATACGACGGCAATACACATTTGAACAGTGTCGAGGCTT
ATGATCCTGAACGTAATGAGTGGTCCTTGGTGGCACCGTTGTCAACGCGCCGTTCCGGGGTAGGGGTAGC
AGTGTTAAATGGCTTGATTTATGCGGTGGGTGGTTATGACGGGAACACCCACTTAAATAGCGTTGAGGCC
TATGATCCGCATCACAACGAATGGAGTCTGGTCGCCCCTCTGAGTACCCGTCGCAGCGGAGTAGGTGTTG
CTGTCTTAAATGGCTTAATTTACGCGGTCGGCGGTTATGATGGAAATACGCACCTGAATTCCGTAGAAGC
TTACGATCCGGAACGCAATGAATGGTCATTAGTGGCGCCATTATCCACGCGTCGCAGCGGAGTAGGCGTG
GCGGTATTAAACGGTCTTATTTACGCAGTAGGGGGGTACGACGGAAATACTCACTTAAACTCCGTAGAGG
CGTATGACCCACATCACAATGAGTGGAGCCTGGTTGCGCCATTGTCTACACGCCGTAGTGGCGTCGGAGT
TGCTGTATTAAACGGGTTAATCTATGCCGTTGGTGGGTACGATGGAAACACACATCTGAATTCAGTGGAG
GCCTATGATCCAGAACGCAACGAATGGTCTCTGGTCGCACCGTTATCTACTCGCCGTAGCGGAGTGGGAG
TAGCAGTTTTGAATGGCTTGATCTACGCTGTGGGCGGTTACGACGGGAATACCCATCTTAACTCTGTCGA
GGCTTACGACCCTCATCACAACGAATGGTCACTGGTGGCACCCTTATCGACTCGTCGCTCTGGTGTGGGG
GTAGCAGTTCTTAATGGCTAACTCGAGTACACGAGG
S6BE-3HH [SEQ ID N0:10] 
MLIYAVGGYDGNTHLNSVEAYDPERNEWSLVAPLSTRRSGVGVAVLNG
LIYAVGGYDGNTHLNSVEAYDPHHNEWSLVAPLSTRRSGVGVAVLNG
LIYAVGGYDGNTHLNSVEAYDPERNEWSLVAPLSTRRSGVGVAVLNG
LIYAVGGYDGNTHLNSVEAYDPHHNEWSLVAPLSTRRSGVGVAVLNG
LIYAVGGYDGNTHLNSVEAYDPERNEWSLVAPLSTRRSGVGVAVLNG
LIYAVGGYDGNTHLNSVEAYDPHHNEWSLVAPLSTRRSGVGVAVLNG
S6BE-L1 [SEQ ID NO:11] 
GATATCCATATGAACGGGCTGATTTATGCCGTTGGAGGCTACGATGGGACAGGGTACAATACCCACCTGA
ATAGCGTAGAGGCTTATGATCCAGAACGTAATGAGTGGTCACTGGTCGCGCCTCTTTCCACGCGCCGCTC
AGGCGTTGGCGTGGCGGTTTTGAACGGATTGATCTACGCGGTTGGTGGTTACGACGGTACTGGCTACAAT
ACTCACTTGAATTCGGTGGAAGCTTATGACCCTGAACGCAATGAATGGAGTTTAGTAGCCCCGCTGAGCA
CGCGTCGCAGTGGCGTCGGCGTTGCGGTTCTTAACGGTTTGATCTATGCGGTCGGAGGATACGACGGGAC
AGGTTACAACACTCATTTAAACTCAGTCGAGGCTTACGATCCAGAGCGCAACGAGTGGTCCTTGGTTGCT
CCCCTTTCAACTCGTCGCTCAGGCGTCGGGGTTGCGGTTTTGAACGGCCTTATTTACGCTGTCGGAGGTT
ACGACGGAACGGGTTATAATACACATCTTAATAGCGTAGAGGCGTATGACCCGGAGCGCAATGAGTGGTC
TCTTGTAGCCCCTCTTAGCACTCGCCGCTCAGGGGTGGGTGTGGCTGTACTGAATGGCCTGATTTATGCA
GTTGGCGGATACGACGGCACGGGGTACAACACACACTTAAACAGTGTGGAAGCCTACGACCCTGAACGCA
ACGAGTGGTCTCTGGTCGCTCCGTTATCGACGCGCCGCTCGGGAGTAGGCGTAGCAGTGCTGAATGGTTT
AATCTACGCTGTGGGAGGATACGATGGCACTGGATACAATACGCATCTGAATTCCGTCGAAGCCTACGAC
CCCGAGCGTAATGAGTGGTCCTTAGTCGCCCCACTTTCGACTCGTCGTTCTGGAGTGGGGGTCGCAGTAT
TATAACTCGAGCGG
S6BE-L1 [SEQ ID NO:12] 
MNGLIYAVGGYDGTGYNTHLNSVEAYDPERNEWSLVAPLSTRRSGVGVAVL
NGLIYAVGGYDGTGYNTHLNSVEAYDPERNEWSLVAPLSTRRSGVGVAVL
NGLIYAVGGYDGTGYNTHLNSVEAYDPERNEWSLVAPLSTRRSGVGVAVL
NGLIYAVGGYDGTGYNTHLNSVEAYDPERNEWSLVAPLSTRRSGVGVAVL
NGLIYAVGGYDGTGYNTHLNSVEAYDPERNEWSLVAPLSTRRSGVGVAVL
NGLIYAVGGYDGTGYNTHLNSVEAYDPERNEWSLVAPLSTRRSGVGVAVL
S6BE-L2 [SEQ ID NO:13] 
GATATCCATATGAATGGATTGATTTACGCCGTAGGTGGTTATGATGGTAGTCCAGATTACTCGACAGGAA
CCCACTTGAATTCGGTAGAAGCCTACGATCCAGAACGTAATGAATGGTCGTTAGTGGCGCCGCTGTCCAC
ACGTCGTTCCGGGGTGGGAGTAGCAGTCTTGAACGGACTTATTTACGCCGTCGGGGGGTATGATGGATCC
CCGGATTACAGTACTGGAACCCACCTGAATAGTGTGGAAGCCTATGATCCCGAGCGCAATGAATGGAGTC
TGGTTGCACCCTTAAGTACCCGTCGTTCCGGCGTCGGAGTAGCAGTATTGAATGGCTTGATCTACGCGGT
AGGAGGGTACGACGGTTCTCCAGACTATAGTACTGGGACTCACCTTAATAGCGTCGAGGCCTACGATCCC
GAACGTAACGAGTGGTCCTTAGTTGCCCCCCTGTCCACACGCCGCTCTGGTGTAGGCGTAGCTGTCTTGA
ACGGGCTTATCTACGCTGTGGGGGGCTATGACGGATCGCCCGATTATTCCACCGGTACGCATTTGAATAG
CGTGGAAGCTTATGACCCAGAGCGTAACGAATGGAGTTTGGTTGCTCCTCTGAGCACCCGTCGCTCTGGG
GTAGGTGTCGCCGTATTGAATGGTTTGATTTATGCAGTTGGCGGCTACGACGGCTCTCCAGATTACAGCA
CTGGCACACACCTGAATTCTGTAGAGGCTTATGATCCGGAGCGCAACGAATGGTCCCTGGTGGCTCCACT
TAGCACACGCCGCAGTGGGGTTGGAGTTGCAGTGTTAAATGGACTGATCTATGCCGTAGGGGGTTACGAT
GGTAGCCCGGACTATTCTACAGGCACCCACCTTAACAGCGTGGAAGCGTACGACCCAGAACGCAATGAGT
GGTCTCTGGTAGCTCCGCTTTCCACACGTCGCTCAGGAGTAGGAGTTGCTGTATTGTAACTCGAGCGG
S6BE-L2 [SEQ ID NO:14] 
MNGLIYAVGGYDGSPDYSTGTHLNSVEAYDPERNEWSLVAPLSTRRSGVGVAVL
NGLIYAVGGYDGSPDYSTGTHLNSVEAYDPERNEWSLVAPLSTRRSGVGVAVL
NGLIYAVGGYDGSPDYSTGTHLNSVEAYDPERNEWSLVAPLSTRRSGVGVAVL
NGLIYAVGGYDGSPDYSTGTHLNSVEAYDPERNEWSLVAPLSTRRSGVGVAVL
NGLIYAVGGYDGSPDYSTGTHLNSVEAYDPERNEWSLVAPLSTRRSGVGVAVL
NGLIYAVGGYDGSPDYSTGTHLNSVEAYDPERNEWSLVAPLSTRRSGVGVAVL
S6BE-L3 [SEQ ID NO:15] 
GATATCCATATGAACGGGCTTATCTATGCTGTTGGCGGTTATGATGGCTCCCCGGATGGGTCTACCCATC
TGAATTCGGTCGAAGCCTATGACCCCGAGCGCAACGAATGGTCCTTAGTCGCCCCGCTGAGTACACGTCG
TAGCGGAGTAGGTGTTGCGGTGCTTAACGGATTGATCTATGCGGTTGGTGGGTTTGCAACTCTGGAAACC
GAATCCGGAGAGTTGGTCCCGACAGAACTTAACAGTGTAGAAGCGTATGACCCTGAACGTAATGAGTGGT
CGTTAGTAGCTCCTTTATCAACGCGCCGTAGTGGGGTTGGAGTTGCAGTTTTAAATGGACTGATCTACGC
AGTAGGAGGATATGATGGGTCGCCTGACGGGAGTACGCACCTTAATTCGGTAGAGGCGTACGATCCAGAG
CGTAACGAGTGGAGTCTTGTAGCGCCACTTTCAACACGTCGTTCGGGTGTCGGAGTGGCCGTTCTGAATG
GGTTAATTTATGCTGTCGGAGGGTTTGCCACACTTGAGACCGAGAGTGGAGAGCTTGTGCCAACGGAGTT
AAACTCAGTGGAAGCTTATGATCCTGAGCGCAATGAGTGGTCACTGGTCGCTCCCCTGAGTACTCGCCGC
AGCGGAGTGGGAGTAGCAGTGTTAAATGGACTGATTTATGCCGTGGGCGGCTATGACGGGAGTCCTGATG
GTAGCACGCATCTGAATTCGGTGGAGGCCTACGATCCCGAGCGTAACGAGTGGTCCCTGGTAGCGCCTCT
TTCGACTCGTCGCTCAGGCGTGGGGGTAGCCGTCTTAAACGGCCTTATTTACGCCGTCGGAGGTTTTGCC
ACCTTAGAGACTGAATCTGGCGAATTAGTCCCAACCGAGCTGAACTCCGTCGAAGCGTATGATCCAGAAC
GCAATGAGTGGTCTTTGGTTGCTCCTCTGTCGACTCGTCGTTCAGGGGTGGGAGTGGCGGTATTATAACT
CGAGCGG
S6BE-L3 [SEQ ID NO:16] 
MNGLIYAVGG YDGSPDGSTH LNSVEAYDPERNEWSLVAPLSTRRSGVGVAVL
NGLIYAVGG FATLETESGELVPTE LNSVEAYDPERNEWSLVAPLSTRRSGVGVAVL
NGLIYAVGG YDGSPDGSTH LNSVEAYDPERNEWSLVAPLSTRRSGVGVAVL
NGLIYAVGG FATLETESGELVPTE LNSVEAYDPERNEWSLVAPLSTRRSGVGVAVL
NGLIYAVGG YDGSPDGSTH LNSVEAYDPERNEWSLVAPLSTRRSGVGVAVL
NGLIYAVGG FATLETESGELVPTE LNSVEAYDPERNEWSLVAPLSTRRSGVGVAVL
S6BR [SEQ ID NO:17] 
GGGAATTCCATATGGGGGGGCTTTTATATGCTGTTGGTGGATATGATGGAAACACTCATCTTAATTCTCT
TGAATGCTATAACCCCGAGACCAACGAATGGAGTCCAGTAGCTCCTCTTTCCGTGCCACGTTCTGGTATT
GGTGTTGCTGTTTTGGGGGGACTTTTGTATGCAGTCGGGGGATACGACGGCAATACCCATCTGAATTCAT
TAGAGTGTTATAACCCTGAGACCAATGAATGGTCTCCAGTAGCTCCTCTGTCAGTGCCACGTTCCGGTAT
CGGGGTAGCGGTGCTGGGTGGTCTGCTGTACGCGGTGGGAGGATACGATGGCAATACGCATTTAAATTCT
TTGGAGTGCTATAACCCCGAAACAAACGAGTGGTCTCCCGTCGCACCCCTGTCGGTACCGAGATCAGGCA
TAGGCGTCGCTGTGCTTGGGGGACTGTTATACGCTGTCGGTGGTTATGATGGAAATACGCACCTTAACAG
CCTTGAATGTTACAATCCGGAAACAAATGAATGGTCCCCGGTAGCTCCCTTGTCGGTCCCCCGTTCGGGC
ATAGGGGTAGCTGTGTTAGGAGGATTGTTGTATGCAGTGGGTGGGTATGACGGTAATACACACCTGAACT
CATTAGAGTGCTATAACCCCGAGACCAACGAGTGGTCGCCAGTTGCTCCCTTGTCAGTTCCCCGGTCCGG
AATCGGGGTCGCTGTCTTGGGAGGGCTTTTGTATGCGGTCGGGGGCTATGATGGTAATACTCACCTGAAT
TCGCTTGAATGCTATAATCCGGAAACTAACGAATGGTCTCCGGTTGCACCTCTGTCGGTACCGCGTAGTG
GCATTGGCGTGGCAGTATTATAACTCGAGCGG
S6BR [SEQ ID NO:18] 
MGGLLYAVGGYDGNTHLNSLECYNPETNEWSPVAPLSVPRSGIGVAVL
GGLLYAVGGYDGNTHLNSLECYNPETNEWSPVAPLSVPRSGIGVAVL
GGLLYAVGGYDGNTHLNSLECYNPETNEWSPVAPLSVPRSGIGVAVL
GGLLYAVGGYDGNTHLNSLECYNPETNEWSPVAPLSVPRSGIGVAVL
GGLLYAVGGYDGNTHLNSLECYNPETNEWSPVAPLSVPRSGIGVAVL
GGLLYAVGGYDGNTHLNSLECYNPETNEWSPVAPLSVPRSGIGVAVL
S6BC [SEQ ID NO:19] 
GGGAATTCCATATGAATGGACACATATATGCAGTTGGTGGCTATGACGGACATACCCATTTAAACTCAGT
TGAGGCTTATGACCCGGAGACCGATGAATGGCGCTTGGTCGCACCGTTGACGACGCCCAGATCCGGTATG
GGAGTTGCTGTTCTTAACGGCCATATATATGCGGTAGGCGGGTATGATGGTCATACCCACCTGAATTCCG
TAGAGGCCTATGATCCAGAGACTGACGAGTGGAGACTTGTTGCCCCTTTAACGACACCACGGTCGGGCAT
GGGGGTCGCCGTCCTTAACGGTCACATATACGCAGTCGGCGGCTATGATGGTCATACCCACCTGAATAGT
GTGGAAGCTTACGATCCAGAAACGGACGAATGGCGCTTAGTTGCTCCGTTGACGACACCCAGAAGCGGGA
TGGGTGTGGCTGTGCTGAACGGCCATATATATGCAGTAGGTGGGTACGATGGACACACACATCTTAATTC
CGTGGAAGCCTATGATCCCGAAACTGATGAGTGGCGGCTTGTAGCTCCACTGACGACCCCACGCAGCGGG
ATGGGGGTAGCTGTTCTGAACGGCCATATTTACGCGGTGGGGGGCTATGACGGCCATACGCATTTAAATT
CTGTCGAGGCATACGACCCAGAGACAGATGAATGGCGCCTGGTAGCACCTCTTACTACACCTAGATCTGG
CATGGGAGTCGCGGTTTTGAATGGGCACATCTACGCCGTGGGGGGCTACGATGGACATACACATTTGAAT
TCTGTCGAAGCTTATGATCCCGAGACAGATGAATGGAGACTGGTTGCCCCATTGACCACGCCCCGGAGTG
GAATGGGCGTAGCTGTACTTTAACTCGAGCGG
S6BC [SEQ ID NO:20]

MNGHIYAVGGYDGHTHLNSVEAYDPETDEWRLVAPLTTPRSGMGVAVL
NGHIYAVGGYDGHTHLNSVEAYDPETDEWRLVAPLTTPRSGMGVAVL
NGHIYAVGGYDGHTHLNSVEAYDPETDEWRLVAPLTTPRSGMGVAVL
NGHIYAVGGYDGHTHLNSVEAYDPETDEWRLVAPLTTPRSGMGVAVL
NGHIYAVGGYDGHTHLNSVEAYDPETDEWRLVAPLTTPRSGMGVAVL
NGHIYAVGGYDGHTHLNSVEAYDPETDEWRLVAPLTTPRSGMGVAVL

Specific embodiments are polypeptides with SEQ ID NO: 2, 4, 6, 8, 10, 12, 14, 16, 18, and 20, wherein the Nterminal methionine is absent.
keap1 [SEQ ID NO:21] 
GRLIYTAGG-----YFRQSLSYLEAYNPSNGTWLRLADLQVPRSGLAGCVV (blade 1)
GGLLYAVGGRNNSPDGNTDSSALDCYNPMTNQWSPCAPMSVPRNRIGVGVI (blade 2)
DGHIYAVGG----SHGCIHHNSVERYEPERDEWHLVAPMLTRRIGVGVAVL (blade 3)
NRLLYAVGG----FDGTNRLNSAECYYPERNEWRMITAMNTIRSGAGVCVL (blade 4)
HNCIYAAGG----YDGQDQLNSVERYDVETETWTFVAPMKHRRSALGITVH (blade 5)
QGRIYVLGG----YDGHTFLDSVECYDPDTDTWSEVTRMTSGRSGVGVAVT (blade 6)
NGLIYAVGG----YDGNTHLNSVEAYDPERNEWSLVAPLSTRRSGVGVAVL [consensus]
[SEQ ID NO:33]
S6AE (S6_2E) [SEQ ID NO: 22]
   DGRIYAVGGYDGSPDGHTHLNSVEAYDPETDTWSLVAPMKTRRSGVGVAVL
S6AR (S6_2R) [SEQ ID NO:23]
   NRLLYAVGGYDGSPDGHTHLNSVECYDPETNEWSLVAPMNTRRSGVGVAVL
S6AC (S6_2C) [SEQ ID NO:24]
   NGRIYAVGGYDGSPDGHTHLNSVEAYDPETDEWSFVAPMTTPRSGVGVAVL
S6BE (S6_6E) [SEQ ID NO:25]
   NGLIYAVGGYDGNTHLNSVEAYDPERNEWSLVAPLSTRRSGVGVAVL
S6BE-3HH (S6_6E-3HH) [SEQ ID NO:26]
   NGLIYAVGGYDGNTHLNSVEAYDPERNEWSLVAPLSTRRSGVGVAVL (blade 1)
   NGLIYAVGGYDGNTHLNSVEAYDPHHNEWSLVAPLSTRRSGVGVAVL (blade 2)
S6BE-L1 (S6_6E-L1) [SEQ ID NO:27]
   NGLIYAVGGYDGTGYNTHLNSVEAYDPERNEWSLVAPLSTRRSGVGVAVL
S6BE-L2 (S6_6E-L2) [SEQ ID NO:28]
   NGLIYAVGGYDGSPDYSTGTHLNSVEAYDPERNEWSLVAPLSTRRSGVGVAVL
S6BE-L3 (S6_6E-L3) [SEQ ID NO:29]
   NGLIYAVGGYDGSPDGSTHLNSVEAYDPERNEWSLVAPLSTRRSGVGVAVL (blade 1)
   NGLIYAVGGFATLETESGELVPTELNSVEAYDPERNEWSLVAPLSTRRSGVGVAVL (blade 2)
S6BR (S6_6R) [SEQ ID NO:30]
   GGLLYAVGGYDGNTHLNSLECYNPETNEWSPVAPLSVPRSGIGVAVL
S6BC (does not express) (S6_6C) [SEQ ID NO:31]
   NGHIYAVGGYDGHTHLNSVEAYDPETDEWRLVAPLTTPRSGMGVAVL
NGRIYAVGGXₙLNSVEAYDPETDEWSFVAPMTTPRSGVGVAVL [SEQ ID NO:32]wherein X n is 1 to 10 or 15 amino acids.

**Table 1: Crystallographic information tables for the 4 initial SAKe designs.**

| | **S6AE** | **S6AR** | **S6AC** | **S6BE (normal)** |
|---|---|---|---|---|
| **PDB code** | 7ON6 | 7ON8 | 7ONA | 7ONC |
| **Wavelength** | 1.00003 | 1.00003 | 1.00003 | 0.92003 |
| **Resolution range** | 38.12 - 1.35 (1.398 - 1.35) | 39.12 - 1.5 (1.554 - 1.5) | 39.77 - 1.3 (1.347 - 1.3) | 23.4 - 1.1 (1.139 - 1.1) |
| **Space group** | P 31 2 1 | P 21 21 21 | I 2 2 2 | P 6 |
| **Unit cell** | 82.767 82.767 90.023 90 90 120 | 45.986 63.249 99.567 90 90 90 | 46.932 74.8725 74.8655 90 90 90 | 46.8027 46.8027 34.9814 90 90 120 |
| **Total reflections** | 1541949 (142973) | 618399 (60166) | 410214 (40479) | 337801 (31921) |
| **Unique** | 78555 (7776) | 47304 (4657) | 32753 (3206) | 17817 (1780) |
| **reflections** | | | | |
| **Multiplicity** | 19.6 (18.4) | 13.1 (12.9) | 12.5 (12.6) | 19.0 (17.9) |
| **Completeness(%)** | 99.99 (100.00) | 99.98 (100.00) | 99.70 (98.86) | 99.78 (99.83) |
| **Mean I/sigma(I)** | 50.48 (6.70) | 26.77 (2.58) | 26.52 (4.59) | 39.80 (12.34) |
| **Wilson B-factor** | 14.63 | 15.39 | 15.69 | 10.9 |
| **R-merge** | 0.03554 (0.5203) | 0.06382 (1.053) | 0.04844 (0.4897) | 0.0397 (0.1398) |
| **R-meas** | 0.03649 (0.535) | 0.06645 (1.097) | 0.05057 (0.5099) | 0.04116 (0.1441) |
| **R-pim** | 0.008191 (0.1242) | 0.01832 (0.3023) | 0.01429 (0.14) | 0.01045 (0.03452) |
| **CC1/2** | 1 (0.966) | 1 (0.818) | 0.999 (0.97) | 0.993 (0.987) |
| **CC**^{∗} | 1 (0.991) | 1 (0.948) | 1 (0.992) | 0.998 (0.997) |
| **Reflections used** | 78552 (7776) | 47302 (4657) | 32741 (3201) | 17809 (1779) |
| **in refinement** | | | | |
| **Reflections used** | 3890 (363) | 2325 (238) | 1535 (137) | 829 (86) |
| **for R-free** | | | | |
| **R-work** | 0.1370 (0.1445) | 0.1763 (0.2070) | 0.1584 (0.2036) | 0.1311 (0.0978) |
| **R-free** | 0.1578 (0.1885) | 0.1822 (0.2214) | 0.1870 (0.2368) | 0.1503 (0.1404) |
| **CC(work)** | 0.968 (0.954) | 0.957 (0.862) | 0.966 (0.975) | 0.955 (0.976) |
| **CC(free)** | 0.968 (0.908) | 0.944 (0.853) | 0.918 (0.948) | 0.952 (0.944) |
| **Number of non-** | 2715 | 2477 | 1271 | 427 |
| **hydrogen atoms** | | | | |
| **macromolecules** | 2322 | 2259 | 1120 | 374 |
| **ligands** | 30 | 0 | 2 | 0 |
| **solvent** | 363 | 218 | 149 | 53 |
| **Protein residues** | 305 | 302 | 149 | 47 |
| **RMS(bonds)** | 0.009 | 0.011 | 0.01 | 0.019 |
| **RMS(angles)** | 1.1 | 1.22 | 1.08 | 1.65 |
| **Ramachandran** | 97.36 | 95.3 | 97.24 | 97.78 |
| **favored** (%) | | | | |
| **Ramachandran** | 2.64 | 4.7 | 2.76 | 2.2 |
| **allowed** (%) | | | | |
| **Ramachandran** | 0.00 | 0.00 | 0.00 | 0.00 |
| **outliers** (%) | | | | |
| **Rotamer outliers** | 0.00 | 0.00 | 0.00 | 0.00 |
| **(%)** | | | | |
| **Clashscore** | 2.67 | 1.38 | 0.93 | 1.36 |
| **Average B-factor** | 19.94 | 18.82 | 21.35 | 14.52 |
| macromolecules | 17.7 | 17.76 | 20.38 | 12.92 |
| solvent | 33.49 | 29.84 | 28.76 | 25.79 |
| ligands | 29.27 | | 14.3 | |
| **Number of TLS** | | 6 | | |
| **groups** | | | | |

**Table 2: Crystallographic information tables for the self-assembled S6BE structure and S6BE loop variants.**

| | **S6BE (self-assembled)** | **S6BEL1** | **S6BEL2** | **S6BEL3** |
|---|---|---|---|---|
| **PDB code** | 7ONE | 7ONG | 7ON7 | 7ONH |
| **Wavelength** | 1.1808 | 0.9763 | 0.9763 | 0.9763 |
| **Resolution range** | 35.04 - 1.2 (1.243 - 1.2) | 37.87 - 1.95 (2.02 - 1.95) | 39.86 - 1.95 (2.02 - 1.95) | 40.55 - 1.55 (1.606 - 1.55) |
| **Space group** | P 6 | P 6 2 2 | P 1 21 1 | P 41 3 2 |
| **Unit cell** | 46.5489 46.5489 35.0445 90 90 120 | 43.725 43.725 72.146 90 90 120 | 79.96 45.4 81.62 90 114.72 90 | 90.668 90.668 90.668 90 90 90 |
| **Total reflections** | 227910 (14062) | 120255 (11712) | 257814 (24890) | 38062 (3616) |
| **Unique reflections** | 13612 (1351) | 3335 (315) | 39042 (873) | 19032 (1809) |
| **Multiplicity** | 16.7 (10.4) | 36.1 (37.2) | 6.6 (6.5) | 2.0 (2.0) |
| **Completeness** (%) | 99.85 (99.27) | 99.88 (99.68) | 79.84 (22.49) | 99.71 (97.31) |
| **Mean I/sigma(I)** | 62.70 (27.66) | 25.91 (8.83) | 7.37 (1.46) | 95.39 (19.84) |
| **Wilson B-factor** | 11.59 | 22.98 | 11.38 | 12.76 |
| **R-merge** | 0.03273 (0.07503) | 0.1254 (0.6093) | 0.1378 (1.456) | 0.003807 (0.03001) |
| **R-meas** | 0.03382 (0.079) | 0.1273 (0.6176) | 0.1495 (1.582) | 0.005383 (0.04244) |
| R-pim | 0.008205 (0.02438) | 0.02115 (0.1001) | 0.05752 (0.6145) | 0.003807 (0.03001) |
| **CC1/2** | 0.999 (0.998) | 0.999 (0.989) | 0.998 (0.782) | 1 (0.996) |
| **CC**^{∗} | 1 (0.999) | 1 (0.997) | 0.999 (0.937) | 1 (0.999) |
| **Reflections used in** | 13624 (1351) | 3332 (314) | 31325 (873) | 19031 (1809) |
| **refinement** | | | | |
| **Reflections used** | 676 (72) | 178 (13) | 1564 (41) | 954 (88) |
| **for R-free** | | | | |
| **R-work** | 0.1542 (0.1665) | 0.1914 (0.1627) | 0.2317 (0.2502) | 0.1493 (0.1498) |
| **R-free** | 0.1652 (0.2125) | 0.2311 (0.2299) | 0.2786 (0.3103) | 0.1594 (0.1583) |
| **CC(work)** | 0.923 (0.954) | 0.930 (0.968) | 0.855 (0.749) | 0.967 (0.947) |
| **CC(free)** | 0.952 (0.910) | 0.952 (0.955) | 0.866 (0.809) | 0.965 (0.920) |
| **non H atoms** | 418 | 391 | 4644 | 977 |
| **macromolecules** | 373 | 363 | 4061 | 817 |
| **ligands** | 0 | 2 | 0 | 5 |
| **solvent** | 45 | 26 | 583 | 155 |
| **Protein residues** | 47 | 50 | 546 | 107 |
| **RMS(bonds)** | 0.01 | 0.01 | 0.003 | 0.01 |
| **RMS(angles)** | 1.25 | 1.06 | 0.49 | 1.17 |
| **Ramachandran** | 97.78 | 95.83 | 97.68 | 98.1 |
| **favored** (%) | | | | |
| **Ramachandran** | 2.22 | 4.17 | 2.32 | 1.9 |
| **allowed** (%) | | | | |
| **Ramachandran** | 0 | 0 | 0 | 0 |
| **outliers** (%) | | | | |
| **Rotamer outliers** | 0 | 0 | 0.48 | 0 |
| **(%)** | | | | |
| **Clashscore** | 0 | 0 | 3.27 | 1.25 |
| **Average B-factor** | 15.29 | 22.68 | 11.65 | 17.67 |
| macromolecules | 13.87 | 22.46 | 10.98 | 15.29 |
| solvent | 27.07 | 25.49 | 16.32 | 29.44 |
| ligands | | 24.76 | | 41 |
| **Number of TLS** | | 1 | 2 | 9 |
| **groups** | | | | |

**Table 3: Crystallographic information tables for the various crystal structures of S6BE-3HH.**

| | **S6BE-3HH** | **S6BE-3HH (self-assembled)** | **S6BE-3HH (alternative packing)** |
|---|---|---|---|
| **PDB code** | 7OP4 | 7OPU | 7OPV |
| **Wavelength** | 1 | 0.9763 | 0.9763 |
| **Resolution range** | 38.15 - 1.52 (1.574 - 1.52) | 68.33 - 1.7 (1.761 - 1.7) | 40.54 - 1.95 (2.02 - 1.95) |
| **Space group** | C 1 2 1 | C 1 2 1 | P 1 |
| **Unit cell** | 44.049 76.29 67.987 90 90.021 90 | 76.4486 44.1528 68.326 90 90.0535 90 | 46.9614 47.1524 70.876 72.1828 76.4037 60.1756 |
| **Total reflections** | 234771 (23764) | 169862 (16090) | 120140 (11837) |
| **Unique reflections** | 33235 (3298) | 25255 (2491) | 34968 (3389) |
| **Multiplicity** | 7.1 (7.2) | 6.7 (6.5) | 3.4 (3.5) |
| **Completeness** (%) | 96.01 (95.04) | 99.79 (99.52) | 96.00 (94.43) |
| **Mean I/sigma(I)** | 30.43 (8.87) | 21.82 (3.29) | 35.05 (14.57) |
| **Wilson B-factor** | 16.85 | 23.2 | 19.29 |
| **R-merge** | 0.03691 (0.207) | 0.0454 (0.5517) | 0.03621 (0.06553) |
| **R-meas** | 0.03988 (0.2231) | 0.04926 (0.6008) | 0.04302 (0.08098) |
| R-pim | 0.01493 (0.08249) | 0.01892 (0.2351) | 0.02296 (0.04661) |
| **CC1/2** | 0.999 (0.99) 1 (0.998) | 1 (0.878) 1 (0.967) | 0.995 (0.29) 0.999 (0.671) |
| **Reflections used in** | 33207 (3293) | 25216 (2479) | 34944 (3388) |
| **refinement** | | | |
| **Reflections used for** | 1644 (153) | 1241 (143) | 1699 (147) |
| **R-free** | | | |
| **R-work** | 0.1855 (0.2014) | 0.1853 (0.2425) | 0.1585 (0.1383) |
| **R-free** | 0.2023 (0.2409) | 0.2256 (0.2483) | 0.2038 (0.2306) |
| **CC(work)** | 0.961 (0.939) | 0.958 (0.872) | 0.966 (0.953) |
| **CC(free)** | 0.947 (0.946) | 0.955 (0.884) | 0.949 (0.866) |
| **Number of non-** | 2320 | 2268 | 4547 |
| **hydrogen atoms** | | | |
| **macromolecules** | 2090 | 2079 | 4113 |
| **ligands** | 0 | 0 | 0 |
| **solvent** | 230 | 189 | 434 |
| **Protein residues** | 280 | 281 | 562 |
| **RMS(bonds)** | 0.006 | 0.006 | 0.007 |
| **RMS(angles)** | 0.93 | 0.85 | 0.79 |
| **Ramachandran** | 97.12 | 97.49 | 96.24 |
| **favored** (%) | | | |
| **Ramachandran** | 2.88 | 2.51 | 3.76 |
| **allowed** (%) | | | |
| **Ramachandran** | 0 | 0 | 0 |
| **outliers** (%) | | | |
| **Rotameroutliers(%)** | 0 | 0 | 0.24 |
| **Clashscore** | 5.16 | 2.72 | 3.38 |
| **Average B-factor** | 20.72 | 26.63 | 21.71 |
| macromolecules | 19.64 | 25.86 | 20.59 |
| solvent | 30.6 | 35.08 | 32.25 |
| ligands | | | |
| **Number of TLS** | | | |
| **groups** | | 6 | 18 |

### Example 1. Protein design

The proteins examined in this research were designed using the RE₃Volutionary protein design method [Voet et al. Proceedings of the National Academy of Sciences 2014, 111, 15102- 15107]. The KELCH domain of human Keap1 (PDB code: 1ZGK) was chosen as a template for the SAKe designs. Clustal Omega was used to generate multiple sequence alignments (MSAs) starting from the six repeats of the keapl *β-*propeller [Madeira et al. Nucleic acids research 2019, 47, W636-W641]. The MSAs and their accompanying unrooted phylogenetic trees were used to construct lists of putative ancestral sequences using the FastML server [The PyMOL Molecular Graphics System, Version 1.2r3pre, Schrodinger, LLC.] , with 250 sequences per node for a total of 10000 sequences for each SAKe construct. Idealized symmetric backbone models were designed using both PyMOL [Ashkenazy et al. Nucleic acids research 2012, 40, W580-W584] and PyRosetta [Chaudhury et al. Bioinformatics 2010, 26, 689-691]. With PyMOL, the second and last blades of the Keap1 *β*propeller were extracted. The first blade was used for the design of type A SAKe (47 amino acids per repeat) and the last blade for type B SAKe (51 amino acids per repeat). The N-termini were truncated to minimize clashing with their symmetry equivalents during the subsequent Rosetta Symmetric Docking procedure [Andre et al. Proc Natl Acad Sci 2007, 104, 17656-17661]. We enforced a sixfold rotational symmetry and generated 20000 Monte Carlo Simulated Annealing (MCSA) optimized models per SAKe type. Results were evaluated on their docking score and RMSD from a manually constructed symmetric backbone. The blades of the best models were reconnected, adding in only the exact amount of amino acids that were removed earlier. The putative ancestral sequences were mapped on their corresponding backbone models using a custom PyRosetta script.For each SAKe type we selected a model with the lowest Talaris2013 energy score (e.g. SAKe6E 'E'), a model with lowest RMSD from the input backbone (e.g. SAKe6R 'R') and a c-optimized model (e.g. SAKe6C 'C'). For all SAKe constructs except S6AR, cysteine residues were mutated to serine or alanine. Following our interpretation of later experiments, S6BE mutants were rationally designed to have altered self-assembly properties. The S6BE- 3HH variant was designed from S6BE via following mutations: E24H-R25H, E118H-R119H and E212H-R213H. Amino acid sequences were reverse translated into DNA sequences, using a codon optimization tool provided by the supplier (Integrated DNA Technologies, Iowa, United States).

### Example 2. Production of proteins

DNA sequences were cloned into pET-28a(+) via NdeI and XhoI restriction sites, adding an N-terminal hexahistidine tag to the constructs. For cloning, the recombinant vectors were transformed into *E. coli* DH5*α* via heatshock. The vectors were validated via Sanger Sequencing (LGC Ltd, Teddington, United Kingdom), using T7 promotor and terminator primers provided by LGC. Correct plasmids were transformed into *E. coli* BL21 via heat shock. 1L cultures were grown in a shaking incubator at 37 °C to an OD₆₀₀ of 0.6. Thereafter, cultures were incubated on ice for 20 min. After adding 1 mM isopropyl *β*-D-1thiogalactopyranoside (IPTG) incubation was continued at 20 °C for 16-18 h while shaking. Cells were harvested via centrifugation at 3000 g. The supernatant was discarded and pellets were immediately stored at -24 °C. Pellets were thawed on ice and suspended in 40 mL of 50 mM NaH₂PO₄ (pH 8), 200 mM NaCl, 10 mM Imidazole, 1 mM phenylmethylsulfonyl fluoride (PMSF) and 30 mg hen eggwhite lysozyme. They were incubated for 30 min at 15 °C, while rotating. After, they were lysed via sonication. Lysates were centrifuged at 3000 g for 30 min. The Supernatant was filtered (0.45 uM) and loaded on a Nickel nitrilotriacetic (NiNTA) column equilibrated with a 50 mM NaH₂PO₄ (pH 8), 200 mM NaCl and 10 mM imidazole buffer. The column was washed with 10 mM and 20 mM imidazole and the proteins eluted with 300 mM imidazole. The fractions containing our proteins were collected and dialyzed overnight in 50 mM NaH₂PO₄ (pH 8) and 200 mM NaCl. At the same time, histidine tags were removed via thrombin (100 U per protein). The dialyzed samples were subjected to an additional Ni-NTA chromatography step and then loaded on a Superdex200pg 16/600 column equilibrated with 20 mM HEPES (pH 8) and 200 mM NaCl.

AbS₂₈₀ peaks were collected, dialyzed in 20 mM HEPES (pH8) and concentrated to stocks of 20 mg/mL or more. These stock proteins were stored at 4 °C. The Superdex column was standardized using the Bio-Rad "gel filtration standard 151-1901" protein marker.

### Example 3. Crystallization and Xray diffraction

Proteins were crystallized via sitting-drop vapor diffusion; using Qiagen Nextal Crystal Screening kits, MRC 96-well plates and an ARI Gryphon robot. For native crystallography droplets consisted of 0.3 µL mother liquor and 0.3 uL of 10 mg/mL protein in 20 mM HEPES pH 8.0, 200 mM NaCl. Protein crystals were vitrified after single-step soaking. PEG 400 or glycerol were used as cryoprotectant. Xray diffraction experiments were performed at Diamond Light Source (United Kingdom), Elletra (Italy) and SLS (Switzerland). The diffraction patterns were indexed using XDS or DIALS [Kabsc Acta Crystallographica Section D: Biological Crystallography 2010, 66, 125-132; Winter et al. Acta Crystallographica Section D. 2018, 74, 85-97]. Data reduction was done with Aimless in CCP4 [Evans et al. Acta Crystallographica Section D: Biological Crystallography 2013, 69, 1204-1214; Winn et al. Acta Crystallographica Section D: Biological Crystallography 2011, 67, 235-242]. Molecular Replacement phasing was done with PHASER, using our computationally designed models as search ensemble [McCoy et al. Journal of applied crystallography 2007, 40, 658-674]. Refinement was done manually with phenix.refine and Coot [Adams et al. Acta Crystallographica Section D: Biological Crystallography 2010, 66, 213-221; Emsley et al. Acta Crystallographica Section D: Biological Crystallography 2010, 66, 486-501]. The final structures were validated using Molprobity and the PDB validation tool [Chen et al. Acta Crystallographica Section D: Biological Crystallography 2010, 66, 12-21], before being deposited at RCSB PDB. The data for S6BE-L2 was severely anisotropic. The Diffraction Anisotropy Server was used to improve the corresponding MTZ file (merged with Aimless at a 1.95 °*A* cutoff).

**Table 4: Crystallization conditions for all SAKe crystals deposited in RCSB PDB.**

| **Protein** | **PDB code Final protein concentration** | | **Reservoir solution** |
|---|---|---|---|
| **S6AE** | 7ON6 | 5 mg/mL | 0.2 M tri-potassium citrate, 1.6 M AmSO4 |
| **S6AR** | 7ON8 | 5 mg/mL | 58% (v/v) MPD, 20% (v/v) Glycerol, 0.085 M |
| | | | HEPES |
| **S6AC** | 7ONA | 5 mg/mL | 0.19 M Calcium chloride, 0.095 M HEPES |
| | | | sodium salt pH 7.5, 26.6% (v/v) PEG 400, |
| | | | 5% (v/v) Glycerol |
| **S6BE** | 7ONC | 5 mg/mL | 0.2 M Potassium formate, 20 %(w/v) PEG |
| | | | 3350 |
| **S6BE (self-assembled)** | 7ONE | 5 mg/mL | 50 mM acetate - acetic acid, pH 4.0 |
| **S6BE-3HH** | 7OP4 | 5 mg/mL | 0.1 M MES pH 6.5, 0.9 M Na phosphate, 0.9 |
| | | | M K phosphate |
| **S6BE-3HH (self-assembled)** | 7OPU | 10 mg/mL | 50 mM citrate - citric acid, pH 4.0 |
| **S6BE-3HH (alternative packing)** | 7OPV | 5 mg/mL | 0.1 M MES pH 6.5, 1.2 M tri-Na citrate, 0.995 mM Cu(NO3)2 |
| **S6BE-L1** | 7ONG | 5 mg/mL | 0.2 M Calcium acetate, 0.1 M Sodium |
| | | | cacodylate pH 6.5, 40% (v/v) PEG 300 |
| **S6BE-L2** | 7ON7 | 5 mg/mL | 0.2 M Magnesium chloride, 0.1 M Tris pH 8.5, |
| | | | 30% (w/v) PEG 4000 |
| **S6BE-L3** | 7ONH | 5 mg/mL | 0.1 M Tris pH 8.0, 2.4 M Ammonium sulfate |

### Example 4. Determination of pI and surface electrostatics

Surface electrostatics were calculated at pH 4.0 and 8.0 via PDB2PQR, using a complete

SAKe monomer as input. The calculated surfaces were visualized via PyMOL. pI values were calculated via PROPKA [Dolinsky et al. Nucleic acids research 2004, 32, W665-W667;Li et al. Proteins: Structure, Function, and Bioinformatics 2005, 61, 704-721;Dolinsky et al. Nucleic acids research 2007, 35, W522- W525; Olsson et al. Journal of chemical theory and computation 2011, 7, 525-537].

### Example 5. CD spectroscopy

CD spectroscopy was performed with a JASCO J-1500 spectrometer. To measure the CD spectra, protein samples were diluted to 400 uL of 0.1 mg/mL in 20 mM NaH₂PO₄ (pH 7.6). Ellipticity was measured at 20 °C from 260 nm to 200 nm, using 1 mm cuvettes. 5 Accumulations were averaged. For determination of melting temperatures, samples were diluted to 400 uL of 0.25 mg/mL in 20 mM NaH₂PO₄ (pH 7.6). The signal at 233 nm was followed from 0 to 95 °C with intervals of 0.2 °C, using sealable 2 mm cuvettes. The data was analyzed with a custom Python script, which fits a sigmoidal curve and extracts its midpoint. The positive signal at 233 nm was previously attributed to interactions between aromatic residues. Disappearance of this signal during melting experiments seemed indicative of tertiary structure disruption [Saxena et al.. Biochemistry 1996, 35, 15215-15221; Nikkhah et al. Biomolecular engineering 2006, 23, 185-194].

**Table 5: Melting temperatures obtained via CD measurements.**

| **Protein** | **PDB code(s)** | **Tm (°C)** |
|---|---|---|
| **keapl** | 1ZGK | 44.1 |
| **S6AE** | 7ON6 | 67.1 |
| **S6AR** | 7ON8 | 75.3 |
| **S6AC** | 7ONA | 87.2 |
| **S6BE** | 7ONC, 7ONE | 95 |
| **S6BE-3HH** | 7OPU, 7OP4, 7OPV | >95 |
| **S6BE-L1** | 7ONG | 60.6 |
| **S6BE-L2** | 7ON7 | 56 |
| **S6BE-L3** | 7ONH | 51.7 |

### Example 6. Spontaneous crystal assembly

The tipping point of pH induced self-assembly was found to be approximately 4.5. To show reversibility of assembly, 500 µL of 5 mg/mL S6BE was dialyzed at 20 °C in 50 mM citrate - citric acid buffer at pH 4.0, 4.5 and 5.0. To confirm the pH 4.5 tipping point, a similar experiment was repeated for both S6BE and S6BE-3HH. 500 uL Samples of various protein concentrations (5.0 mg/mL, 2.5 mg/mL, 1.0 mg/mL and 0.5 mg/mL) were dialyzed at 20 °C in 50 mM citrate - citric acid buffer (pH 4.5 and 4.0). For each protein, a self-assembled crystal was soaked in cryo-protectant, vitrified and shipped of for Xray diffraction. Pictures of the self-assembled crystals were taken with a Nikon SMZ800N microscope, outfitted with a TV Lens C 0.45x (Nikon, Japan).

### DLS

Concentrated Sake6 and Sake6-3HH protein stock solutions (20 mg/ml, HEPES pH 8) were diluted with either 20 mM MES pH 5.6 or MilliQ to a concentration of 1 mg/ml. Metal suspensions (*Cu*(*NO*₃)₂ and *Zn*(*NO*₃)₂, MilliQ) were titrated into the 100 *µ*l of prepared protein solution to achieve the desired ratio of protein:metal. Size measurements were obtained at 25 °C using a Zetasizer Nano ZS instrument (Malvern Instruments) and quartz cuvette (ZEN2112). Data analysis was performed using the Zetasizer software 7.11 (Malvern Instruments).

### AFM

The protein stock solutions (40 mg/ml, HEPES pH 8) were diluted with the imaging buffer (20 mM MES pH 5.6 or MilliQ) to a desired concentration. The metal salts were suspended in MilliQ (*Cu*(*NO*₃)₂ and *Zn(NO*₃)₂, Sigma-Aldrich) and mixed with the protein solutions to obtain the correct ratio of protein:metal, before being left to incubate for 20 minutes. Next, 30 *µ*l of diluted protein/metal solution was drop cast onto freshly cleaved substrates, muscovite mica (Agar Scientific) or HOPG (ZYB grade, Advanced Ceramics Inc.).

An additional 30 *µ*l droplet of the protein/metal solution was then carefully pipetted onto the AFM's cantilever holder, previously loaded with Nanoworld ARROW-UHF AuD20 (Resonance Frequency 0.7 - 2.0 MHz) and brought into contact with the surface droplet in the AFM. The sample and AFM system was then left to equilibrate for 1 hour at 25 °C before imaging.

The images were captured on a Cypher ES atomic force microscope (Asylum Research) using the amplitude modulation mode whilst in solution. The imaging force and frequency were both carefully adjusted to reduce any disruption in the self-assembled surface arrays. The AFM data processing was performed with a combination of SPIP and Gwyddion software. The imaging buffer was either 20 mM MES (pH 5.6) or MilliQ, with protein concentrations of 0.5 - 5 *µ*M and *Cu*(*NO*₃)₂/*Zn*(*NO*₃)₂ concentrations (5 *µ*M - 50 *µ*

## Claims

1. A polypeptide comprising a sequence having at least 60% identity with NGRIY₅AVG₈G₉-Xₙ-LNSVE₁₄AY₁₆DP₁₈ETDEW₂₃SFVAPM₂₉TTPR₃₃SGVG₃₇VAV₄₀L [SEQ ID NO:32] or comprising 2 to 9 repeats of said sequence, wherein Xₙ are between 1 and 15 amino acids, wherein x can be any amino acid,
and wherein the amino acids Tyr₅, Gly₈, Glyg, Tyr₁₆, Trp₂₃, Arg₃₃, and Val₄₀ in said sequence are conserved.

2. The polypeptide according to claim 1, wherein the amino acids Tyr₅, Gly₈, Glyg, GlU₁₄, Tyr₁₆, Pro₁₈, Trp₂₃, Met₂₉, Arg₃₃, and Gly₃₇ and Val₄₀ in said sequence are conserved.

3. The polypeptide according to claim 1 or 2, comprising between 2 to 9 repeats of said sequence, or comprising 2, 3 or 6 repeats of said sequence.

4. The polypeptide according to any one of claims 1 to 3, wherein said sequence has at least 70, 80, 90 or 95% identity with SEQ ID NO:32 or is identical to SEQ ID NO:32.

5. The polypeptide according to any one of claims 1 to 4, wherein Xₙ are between 5 to 15 amino acids, or between 5 to 10 amino acids.

6. The polypeptide according to any one claims 1 to 5, wherein one or more of said repeats are separated from each other with 1 up to 5 amino acids.

7. The polypeptide according to any one of claims 1 to 6, wherein one or more of said repeats are immediately adjacent to each other.

8. The polypeptide according to any one of claims 1 to 7, wherein all of said repeats are immediately adjacent to each other.

9. The polypeptide according to any one of claims 1 to 8, wherein the repeats of said sequence are identical, with exception of the amino acids Xₙ.

10. The polypeptide according to any one of claims 1 to 9, comprising a first repeat and a second repeat of said sequence wherein said first and second repeat occur alternating in said polypeptide.

11. A polypeptide which is a multimer of non-covalently bound polypeptides as defined in any one of claims 1 to 10.

12. The polypeptide according to claim 11, which is a hexamer of 3 non non-covalently bound polypeptides as defined in any one of claims 1 to 9 having two repeats, or which is a hexamer of 2 non non-covalently bound polypeptides as defined in any one of claims 1 to 9 having 3 repeats.

13. A method of producing a functional protein comprising the steps of:
a) providing a polypeptide according to any one of claims 1 to 12,
wherein Xₙ is random selected or designed by an in silico method,
b) generating a multimeric protein of said polypeptides,
c) testing the multimeric protein for the function, and
d) selecting the multimer protein with the function.

14. The method according to claim 13, wherein the function is protein binding, an enzymatic activity, or the binding to an organic molecule.

15. The method according to any one of claims 13 or 14, further comprising the step of determining whether the multimeric protein is stable at pH below 4, or wherein the multimeric protein is resistant against proteolytic degradation.
